(19) Europäisches Patentamt
European Patent Office
Office européen des brevets

(11) **EP 3 984 549 A1**

(12) **EUROPEAN PATENT APPLICATION**
published in accordance with Art. 153(4) EPC

(43) Date of publication:
**20.04.2022 Bulletin 2022/16**

(51) International Patent Classification (IPC):
*A61K 39/00* (2006.01)   *A61P 35/00* (2006.01)
*A61K 35/17* (2015.01)   *A61K 35/545* (2015.01)

(21) Application number: **20822023.6**

(22) Date of filing: **12.06.2020**

(52) Cooperative Patent Classification (CPC):
**A61K 35/17; A61K 35/545; A61K 39/00;**
**A61P 35/00**

(86) International application number:
**PCT/JP2020/023308**

(87) International publication number:
**WO 2020/251046 (17.12.2020 Gazette 2020/51)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **14.06.2019   JP 2019111293**

(71) Applicants:
• **Thyas Co. Ltd.**
  **Kyoto-shi, Kyoto 606-8304 (JP)**
• **Kyoto University**
  **Kyoto-shi, Kyoto 606-8501 (JP)**

(72) Inventors:
• **YASUI Yutaka**
  **Kyoto-shi Kyoto 604-8381 (JP)**
• **HITOSHI Yasumichi**
  **Kyoto-shi Kyoto 606-8304 (JP)**
• **UENO Hiroyuki**
  **Kyoto-shi Kyoto 606-8304 (JP)**
• **KANEKO Shin**
  **Kyoto-shi Kyoto 606-8501 (JP)**

(74) Representative: **Zellentin & Partner mbB**
**Patentanwälte**
**Rubensstraße 30**
**67061 Ludwigshafen (DE)**

(54) **MEDICINAL COMPOSITION**

(57)   The present invention provides a medicinal composition containing a T cell population that exhibits an excellent antigen specificity and has genetic diversity, and a method for preventing or treating cancer using the medicinal composition.

Figure 12

**Description**

**TECHNICAL FIELD**

The present invention relates to a pharmaceutical composition containing a T cell population with excellent antigen specificity and genetic diversity, and a method for preventing or treating cancer using the pharmaceutical composition.

**BACKGROUND TECHNOLOGY**

T cells play a central role in immune response to abnormal cells such as foreign pathogens, like bacteria or virus, or cancer cells. Accordingly, it is considered that decrease in function of T cells causes pathogenic infection and cancer. Replenishment or regeneration of T cells can be an extremely effective means for ameliorating or treating the diseases caused by decrease in function of T cells.

In studies using human and mice, when performing T cell replenishment therapy for infectious diseases and cancer, a multilateral immune response to target tissue or antigen can be induced with a method using a T cell population having genetic diversity. In addition, it is known that high therapeutic effects can be achieved, because the method is less likely to be affected by decrease in expression of antigen and immune escape due to mutation.

Furthermore, T cell replenishment therapy using T cells proliferated via pluripotent stem cells such as iPS cells has been proposed. It is possible to produce regenerated T cells exhibiting the same antigen specificity as original T cells by using T cells specific to target antigens as raw materials for producing the iPS cells (non-patent document 1, which is incorporated herein by reference in its entirety). According to this method, established iPS cells are initialized in a cell population (colony) unit derived from single cells. It becomes possible with the method to use an iPS cell line which is confirmed to be stable in cell properties, to have good differentiation efficiency to target cells or tissue, and to have no mutation or abnormality by means such as gene analysis. It is considered that in order to confirm quality of clones and their compatibility with patients, clones need to be tested one by one and cloning is therefore an important process.

In the prior art, although gene-modified T cells transfected with a T cell receptor (TCR) or a chimeric antigen receptor (CAR) have been reported, the gene sequence of the receptor to be transfected is single (patent documents 1 to 3). In addition, although a method for producing T cells having the same antigen specificity as the original T cells by re-differentiating iPS cells produced from T cells of peripheral blood has been reported, addition of genetic diversity to them is not mentioned (patent document 4).

**PRIOR ART DOCUMENTS**

**PATENT DOCUMENTS**

    1. Japanese patent publication No. 2017-534261
    2. Japanese patent publication No. 2017-530694
    3. Japanese patent publication No. 2018-514204
    4. Japanese patent publication No. 2017-158559

**NON-PATENT DOCUMENT**

    1. Nishimura T, et al. Generation of rejuvenated antigen-specific T cells by reprogramming to pluripotency and redifferentiation. Cell Stem Cell. 2013; 12:114-126.

**SUMMARY OF THE INVENTION**

**PROBLEMS TO BE SOLVED BY THE INVENTION**

IPS regenerated T cell therapy is a method wherein iPS cells (T-iPS cells) are produced from T cells of an individual patient, the T cells are then regenerated using the T-iPS cells, and the regenerated T cells are utilized for treating the patient.

Usually, in a method utilizing an iPS cell line, cloning is performed, which means removing other clones except the selected one. Accordingly, when the iPS cells established from T cells are cloned, the genetic diversity present in the original T cell population is completely lost. This means that only an immune reaction targeting a single antigen epitope can be used. However, in an immune reaction targeting a single antigen epitope, problems arise in that it is affected easily by decrease in expression of antigen molecules or immune escape due to mutation, and high therapeutic effects can hardly be achieved in the T cell replenishment therapy. On the other hand, in a conventionally performed T cell replenishment therapy, although a T cell population having genetic diversity can be used, there are problems of exhaustion of T cells due to *in-vitro* proliferation of T cell populations, and decrease in immune response to antigens.

A T cell replenishment therapy which can overcome the above-mentioned problems is clinically desired. It is demanded to provide T cell populations that maintain immune response to antigens and have genetic diversity, and an effective method for preventing or treating cancer using the T cell populations.

The purpose of the present invention is to provide a pharmaceutical composition containing a T cell population having excellent antigen specificity and genetic diversity, and a method for preventing or treating cancer using the pharmaceutical composition.

## MEANS FOR SOLVING THE PROBLEMS

The purpose of the present invention is achieved by the following inventions.

[1] A pharmaceutical composition containing T cells as active ingredient, wherein the T cells are characterized by

(i) having specificity to cancer vaccine antigen,
(ii) being CD3 and CD45 positive, and
(iii) producing IFN-$\gamma$ (interferon $\gamma$).

[2] The pharmaceutical composition according to [1], wherein the cancer vaccine antigen is one or more selected from the group consisting of GPC3, WT1, XAGE 1, LMP2, and neoantigens.

[3] The pharmaceutical composition according to [1], wherein the cancer vaccine antigen is GPC3.

[4] The pharmaceutical composition according to [1], wherein the T cells are $\alpha\beta$ T cells, $\gamma\delta$ T cells, helper T cells, cytotoxic T cells, natural killer (NK) T cells, or a mixture thereof.

[5] The pharmaceutical composition according to [1], wherein the T cells are cytotoxic T cells.

[6] The pharmaceutical composition according to [5], wherein the cytotoxic T cells are the T cells obtained by collecting T cells from a patient with cancer inoculated with a cancer vaccine, fractionating cytotoxic T cells for the cancer vaccine antigen contained in the cancer vaccine, and proliferating the fractionated cytotoxic T cells.

[7] The pharmaceutical composition according to [6], characterized in that the cytotoxic T cells are fractionated from the T cells by using one or more steps selected from the group consisting of a step of selecting T cells which express CD137, a step of selecting T cells which produce IFN-$\gamma$, and a step of selecting T cells to which antigen peptide-HLA complex binds.

[8] A pharmaceutical composition according to [6], characterized in that the cytotoxic T cells are proliferated in the presence of one or more cytokines selected from the group consisting of IL-2, IL-7, IL-15, and IL-21.

[9] The pharmaceutical composition according to [5], wherein the cytotoxic T cells are regenerated T cell populations obtained by inducing redifferentiation from iPS cells to the cytotoxic T cells.

[10] The pharmaceutical composition according to [9], wherein the iPS cells are produced from the T cells having specificity to the cancer vaccine antigen by bringing one or more cancer vaccine antigens selected from the group consisting of GPC3, WT1, XAGE 1, LMP2, and neoantigens into contact with the T cells.

[11] The pharmaceutical composition according to [9], wherein the iPS cells are produced from the T cells having specificity to GPC3 by bringing the cancer vaccine antigen GPC3 into contact with the T cells.

[12] The pharmaceutical composition according to [10] or [11], wherein the T cells are collected from the peripheral blood of a patient with cancer or a healthy subject.

[13] The pharmaceutical composition according to [10] or [11], wherein the T cells are collected from the peripheral blood of a patient with cancer.

[14] The pharmaceutical composition according to [9], wherein the redifferentiation comprises fractionating CD8$\alpha$/CD8$\beta$ double-positive T cells by using a flow cytometer.

[15] The pharmaceutical composition according to [9], further comprising culturing for maturation of the re-differentiated T cells with phytohemagglutinin (PHA) and feeder cells of autologous or other house.

[16] The pharmaceutical composition according to any one of [1] to [15], wherein the T cells are mature CD8$\alpha$/CD8$\beta$ double-positive T cells.

[17] The pharmaceutical composition according to any one of [1] to [16], wherein the expression ratio of CD3 of the T cells is 70% or more.

[18] The pharmaceutical composition according to any one of [1] to [17], wherein the T cells have the capability to produce IFN-γ/IL-2.

[19] The pharmaceutical composition according to any one of [1] to [18], wherein the survival rate of the T cells is 60% or more.

[20] The pharmaceutical composition according to any one of [1] to [19], further comprising a pharmaceutically acceptable additive.

[21] The pharmaceutical composition according to [20], wherein the additive is one or more selected from the group consisting of a cell culture medium, a physiological saline solution, and a buffer solution.

[22] The pharmaceutical composition according to any one of [1] to [21], characterized by being used in combination with a cancer vaccine.

[23] The pharmaceutical composition according to any one of [1] to [21], characterized by being administered simultaneously with a cancer vaccine.

[24] The pharmaceutical composition according to any one of [1] to [21], characterized by being administered before or after the administration of a cancer vaccine.

[25] The pharmaceutical composition according to any one of [22] to [24], wherein the cancer vaccine antigen contained in the cancer vaccine is one or more selected from the group consisting of GPC3, WT1, XAGE 1, LMP2, and neoantigens.

[26] The pharmaceutical composition according to any one of [22] to [24], wherein the cancer vaccine antigen contained in the cancer vaccine is GPC3.

[27] The pharmaceutical composition according to any one of [1] to [26], containing $1 \times 10^6$ or more T cells as the amount of one dose.

[28] An anticancer agent comprising the pharmaceutical composition according to any one of [1] to [27].

[29] The pharmaceutical composition according to any one of [1] to [27] for prevention or treatment of cancer.

[30] The pharmaceutical composition according to [29], wherein the cancer is selected from the group consisting of ovarian cancer, hepatoblastoma, hepatocellular cancer, gastric cancer, esophageal cancer, pancreatic cancer, renal cell cancer, breast cancer, malignant melanoma, non-small cell lung cancer, cervical cancer, glioblastoma, prostate cancer, neuroblastoma, chronic lymphocytic leukemia, colorectal cancer, colon cancer, and B-cell non-Hodgkin's lymphoma.

[31] A method for preventing or treating cancer in a patient with cancer, characterized by administering an effective amount of the pharmaceutical composition according to [30] to the patient with cancer.

[32] The method for preventing or treating cancer according to [31], wherein the administration is a single administration or multiple administrations.

## EFFECTS OF THE INVENTION

According to the present invention, a pharmaceutical composition containing T cells having excellent specificity to cancer vaccine antigens and genetic diversity can be provided. By using the T cells of the present invention as an active ingredient in combination with a cancer vaccine, it becomes possible to provide an excellent method for preventing or treating cancer as a T cell replenishment therapy to cancer.

## BRIEF DESCRIPTION OF THE DRAWINGS

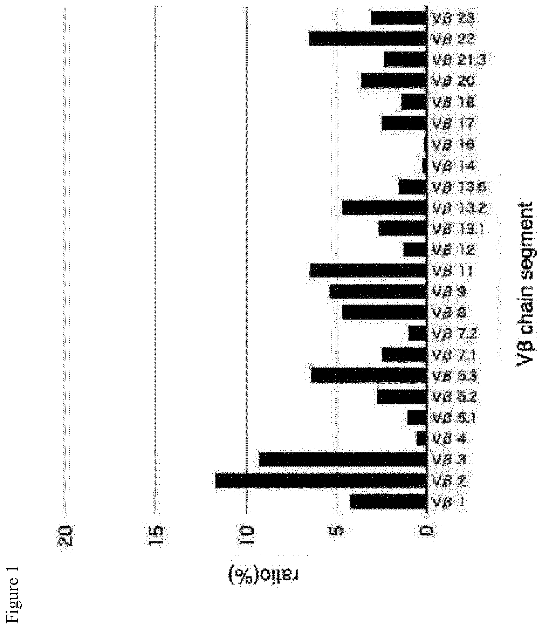

FIG. 1 shows the ratios of T cells having a Vβ chain of each TCR among T cell populations separated from an extracted tumor of a patient with lung cancer. Vβ1 to Vβ23 in the figure indicate that the separated T cell population has genetic diversity of TCR.

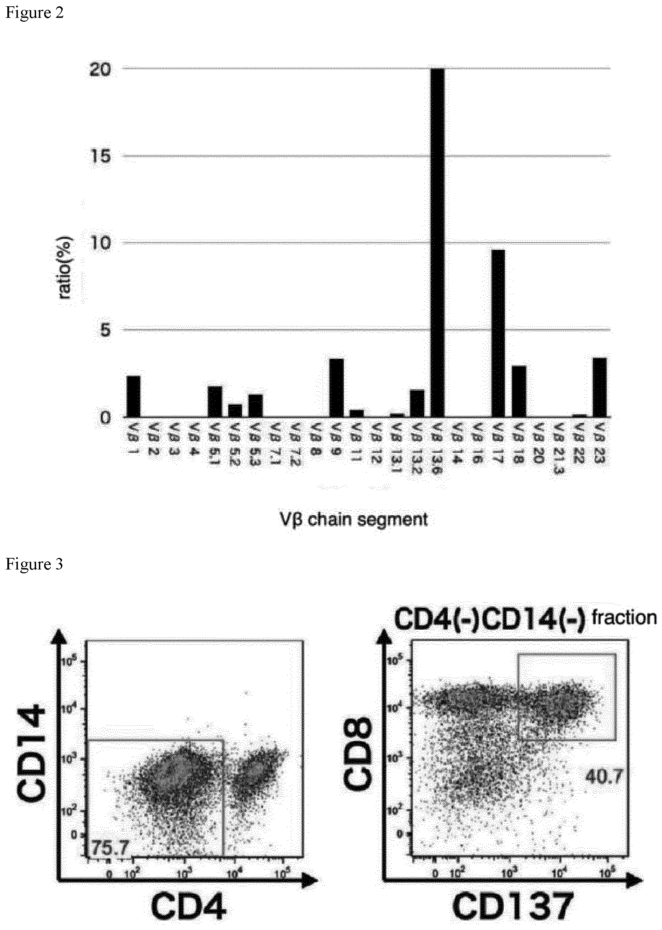

FIG. 2 shows the ratios of T cells having a Vβ chain of each TCR among regenerated T cell populations produced via iPS cells. Vβ1 to Vβ23 in the figure indicate that the regenerated T cell population also has genetic diversity.

FIG. 3 shows the results obtained by culturing for proliferation with GPC3 peptide and analyzing a T cell population expressing an activation marker by a flow cytometer. A fraction which did not express any of CD4 or CD14 (left figure) was gated and developed with CD8 and CD137 (right figure). It is indicated by the figures that CD8/CD137 double-positive cells having directivity to GPC3 were proliferated at a high ratio.

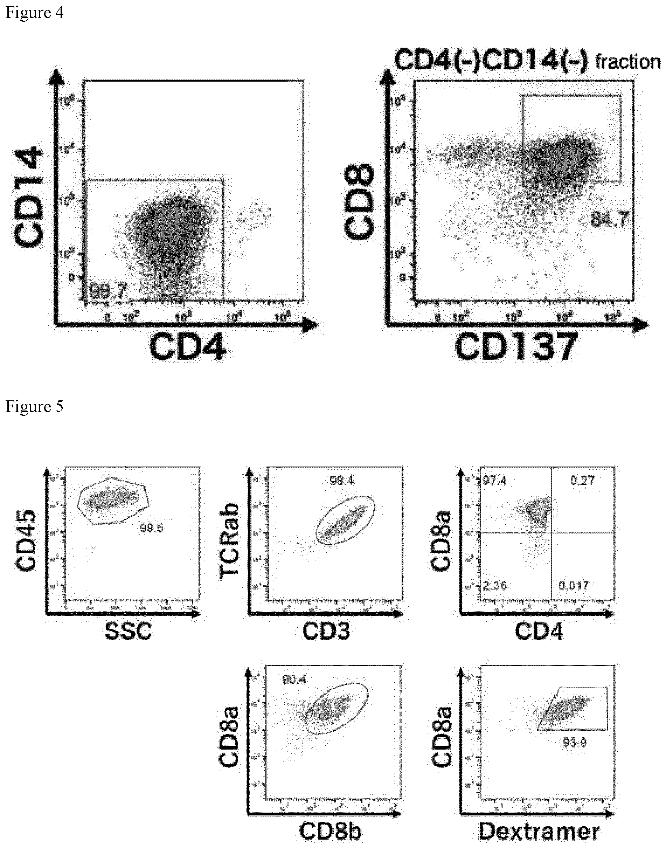

FIG. 4 shows the results obtained by culturing for proliferation with GPC3 peptide, concentrating a cell population expressing both CD8 and CD137 for a T cell population expressing activation marker, and analyzing by a flow cytometer in the same manner as in FIG. 3. It is indicated that the cells expressing the CD4 have been removed by the CD8 negative selection (left figure) when compared to FIG. 3. Besides, it is indicated that the cells not expressing CD137 have also been removed by the CD137 positive selection (right figure). Therefore, it is indicated that CD8/CD137 double-positive cells having directivity to GPC3 were concentrated at a high ratio.

FIG. 5 shows the results of redifferentiation induction of iPS cells initialized from a T cell population having directivity to GPC3 into CD8 positive T cells. The cells with which the redifferentiation induction was performed were stained with antibodies (CD3-APC/Cy7, CD4-BV421, CD8a-PerCP/Cy5.5, CD8b-PE, TCRab-FITC, CD45-BV510, and GPC3-HLA complex (Dextramer)-APC) and analyzed by a flow cytometer (BD FACSAria® II). It is indicated that the re-differentiated CD8 positive T cells were CD8a/CD8b double-positive and bound to GPC3Dextramer at a high ratio.

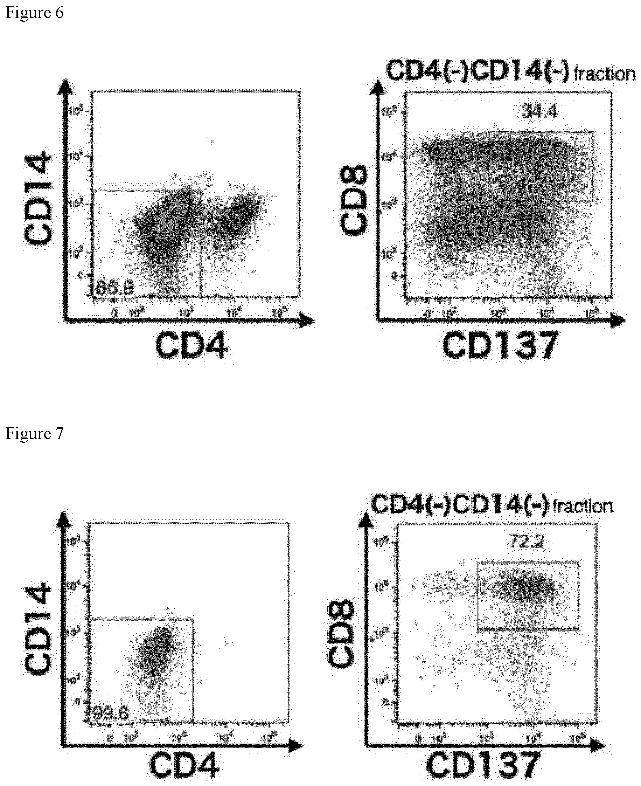

FIG. 6 shows the results obtained by adding WT1 overwrap peptide to mononuclear cells of the peripheral blood of a patient with lung cancer to culture for proliferation, and analyzing the T cell population expressing activation marker in the same manner as in FIG. 3. It is indicated by the figure that CD8/CD137 double-positive cells having directivity to WT1 were proliferated at a high ratio.

FIG. 7 shows the results obtained by culturing for proliferation with WT1 overwrap peptide, and analyzing the T cell population expressing activation marker in the same manner as in FIG. 4. It is indicated by the figure that CD8/CD137 double-positive cells having directivity to WT1 were concentrated at a high ratio.

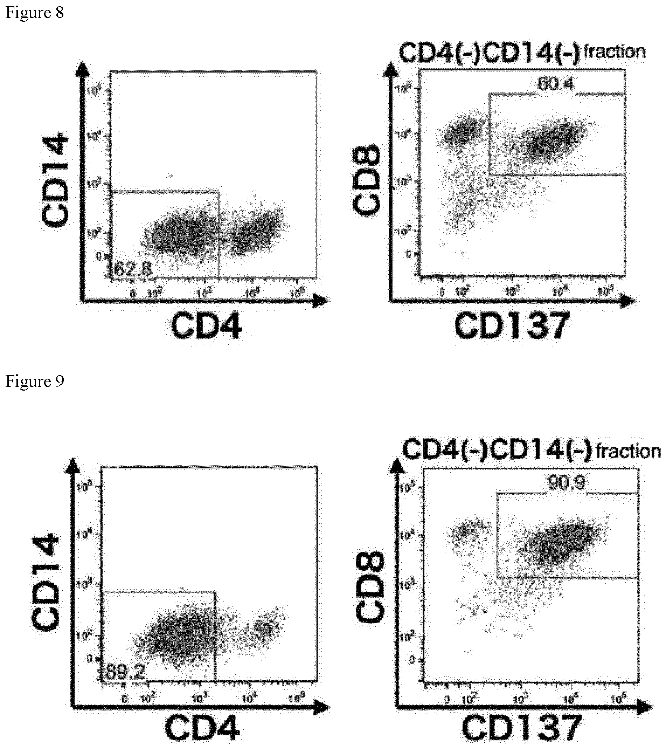

FIG. 8 shows the results obtained by culturing for proliferation mononuclear cells of the peripheral blood of a patient without cancer (healthy subject) using EBV LMP2A overwrap peptide, and analyzing the T cell population expressing activation marker in the same manner as in FIG. 3. It is indicated by the figure that CD8/CD137 double-positive cells having directivity to EBV LMP2A were proliferated at a high ratio.

FIG. 9 shows the results obtained by culturing for proliferation with EBV LMP2A overwrap peptide, and analyzing the T cell population expressing activation marker in the same manner as in FIG. 4. It is indicated by the figure that CD8/CD137 double-positive cells having directivity to EBV LMP2A were concentrated at a high ratio.

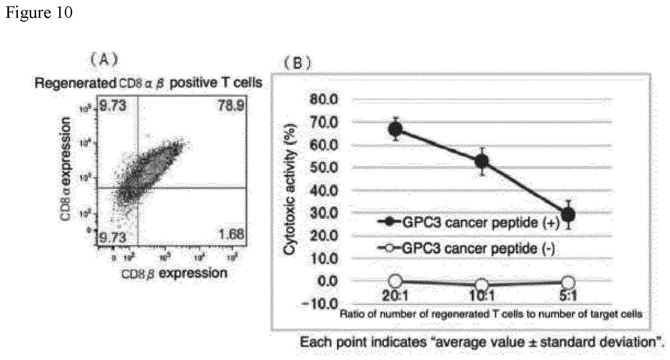

FIG. 10 shows that a regenerated T cell population obtained by inducing for redifferentiation of iPS cells initialized from a T cell population having directivity to GPC3 to CD8 single-positive T cells has a specific cytotoxic activity to target cells expressing GPC3 peptide. (A) shows the analytical results of the regenerated T cell populations by a flow cytometry. (B) shows the results of comparing the cytotoxic activity of the regenerated T cell populations in target cells expressing or not expressing GPC3 peptide.

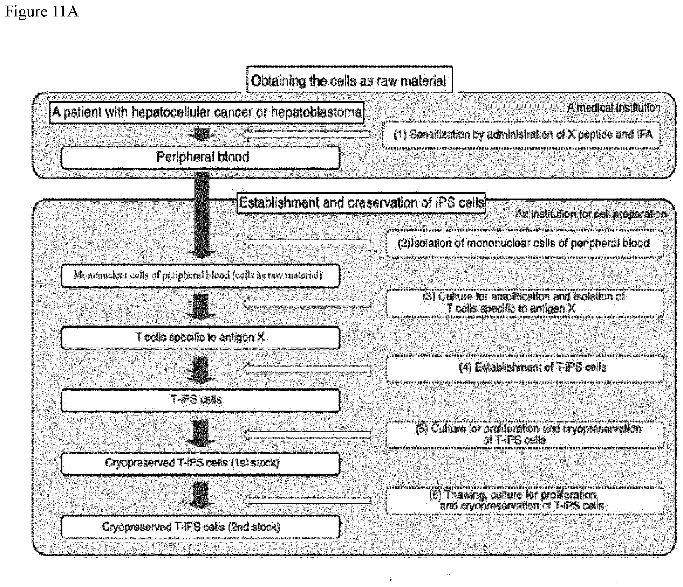

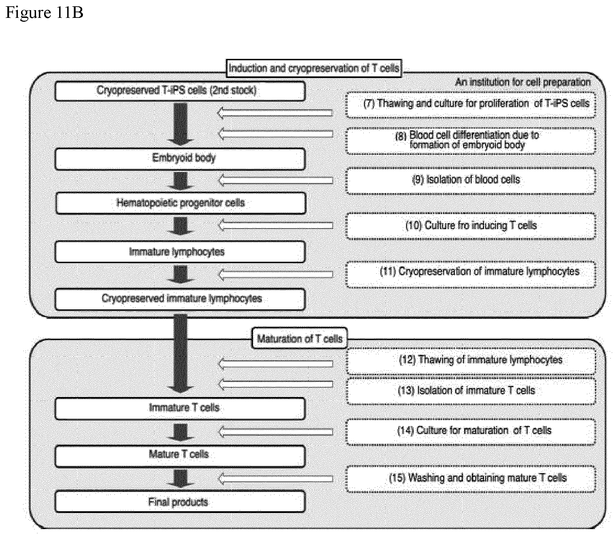

FIG. 11 shows the process for producing the regenerated T cell products.

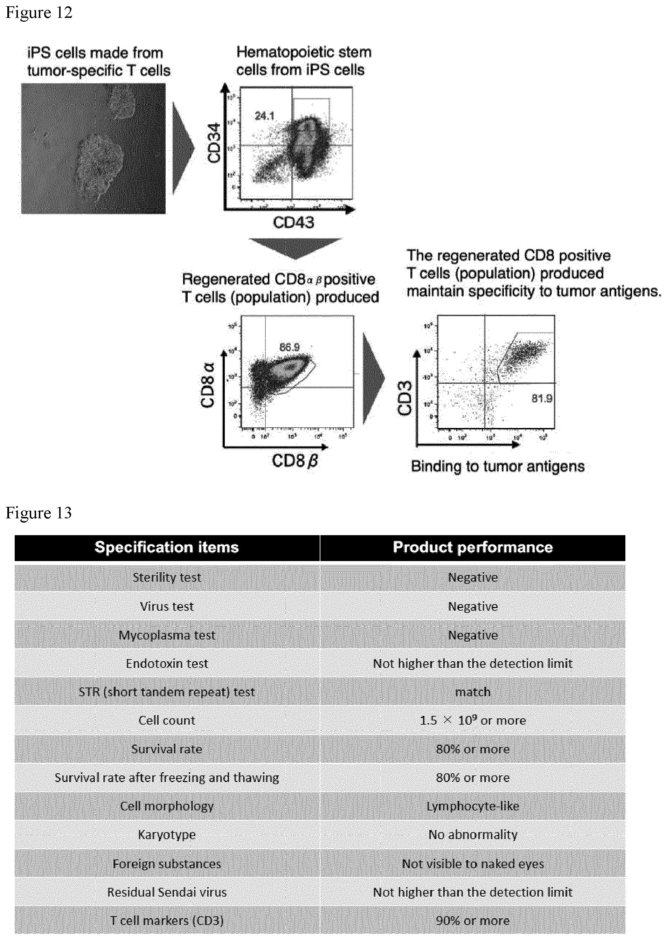

FIG. 12 shows the process for producing regenerated CD8$\alpha$/CD8$\beta$ double-positive T cells from iPS cells derived from tumor antigen-reactive T cells (tumor-specific T cells).

FIG. 13 shows the target values set as the specification for the cell products of the present invention.

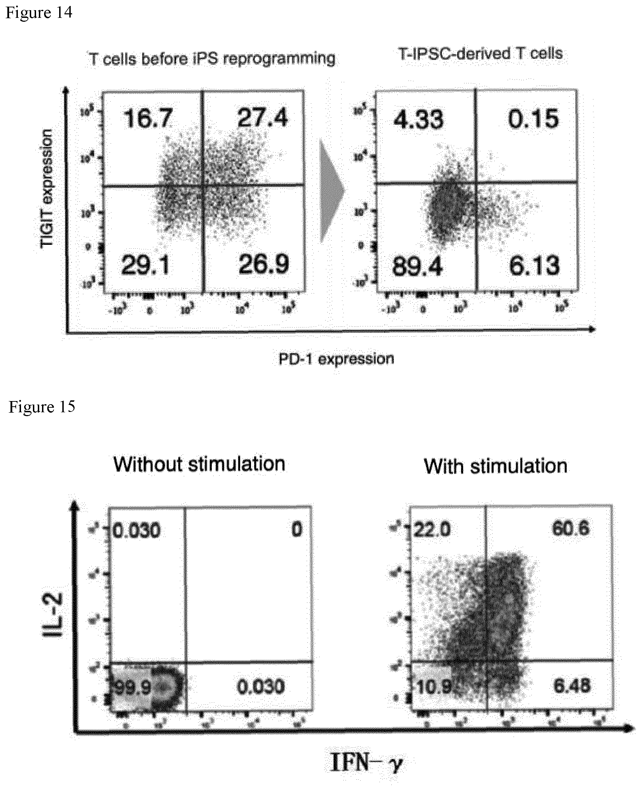

FIG. 14 shows the analytical results of expression of the cell exhaustion marker in T cell populations cultured for proliferation with GPC3 peptide (left) and CD8 positive cells induced for re-differentiation via initialization to iPS cells (right). Each cell was stained with antibodies (TIGIT-Pacific Blue, PD-1-APC/Cy7), and analyzed by using a flow cytometer (BD FACSAria® II). It is indicated that the expression ratio of the cell exhaustion marker is decreased via the initialization to iPS cells.

FIG. 15 shows the results that the regenerated T cells of the present invention have the capability to produce IFN-$\gamma$/IL-2. The antigen on the surface of the regenerated T cells with or without stimulation was stained with antibodies (CD45-BV510, CD8$\alpha$-PerCP/Cy5.5, CD8$\beta$-APC, CD19-FITC), immobilized, and permeated through a cell membrane. After the penetration, it was stained with cytokine antibodies (IFN-$\gamma$-APC/Cy7, IL-2-PE), and the ratio of the regenerated T cells producing cytokine was analyzed by using a flow cytometer (BD FACSAria® II). It is suggested that the regenerated T cells subjected to the stimulation produced IFN-$\gamma$/IL-2 at a high ratio.

## EMBODIMENT FOR CARRYING OUT THE INVENTION

A "cancer vaccine" is a composition comprising "cancer vaccine antigen" for inducing immune response specific for cancer or tumor, which is a cancer or tumor-specific protein or peptide and is derived from cancer or tumor-related antigens. A cell vaccine such as dendritic cells antigen-pulsed with a cancer tissue, protein or peptide that is an antigen specific to cancer or tumor, a viral DNA or RNA vaccine that expresses antigen in an administered living body, and a composition comprising cancer or tumor cells in which proliferation is suppressed, and a crushed material or a lysate thereof are also included in the "cancer vaccine". Furthermore, non-specific immunopotentiators such as bacterial cells, bacterial extract, or $\beta$-glucan, and tumor-soluble virus such as adenovirus are also included in the "cancer vaccine".

A cancer vaccine normally contains adjuvant for enhancing immune response. After being administered to a living body, vaccine antigen contained in a cancer vaccine is phagocytosed by antigen-presenting cells (mainly dendritic cells and macrophages), treated inside the cells, and turned into an epitope peptide comprising amino acids of about 8 to 30 residues, and presented on the surface of antigen-presenting cells as a complex combined with major histocompatibility complex (MHC) class I or class II. The length of the epitope peptide is amino acids of 8 to 11 residues in the case of MHC class I, and amino acids of 13 to 30 residues in the case of MHC class II. The T cell receptor (TCR) expressing on the surface of cytotoxic T cells and helper T cells specifically recognizes MHC class I/peptide complex or MHC class II/peptide complex, respectively. As a result, these T cells are activated to exert antitumor effect. That is, the cytotoxic T cells recognize and destroy cancer cells presenting the same epitope peptide as that contained in the vaccine antigen. The helper T cells enhance the function of cytotoxic T cells through secretion of cytokines and chemokines such as interferon (IFN)-y and interleukin (IL)-2. The helper T cells also have the capability to present antigen-presenting cells via CD40 ligand (CD40L)/CD40 pathway and enhance the production of antigen-specific immunoglobulin (Ig) G antibody in B cells.

In the present invention, examples of "cancer vaccine antigens" for inducing cancer or tumor-specific immune responses include preferably, but are not limited to, tumor-related antigen such as GPC3, WT1, XAGE 1, LMP2, and neoantigens due to gene mutation and abnormality in splicing, more preferably GPC3. The "neoantigen" is an antigen produced from a gene wherein mutation or abnormality in splicing has occurred. A large amount of gene mutation is accumulated in cancer cells. The mutation site and mutation degree are different in case of each patient. In addition, since it is a non-self antigen that does not normally exist in-vivo, T cells can strongly damage cancer cells expressing neoantigens. In the present invention, neoantigen is referred to those which are used as a vaccine obtained by testing a gene mutation or abnormality in splicing contained in cancer cells for each patient, synthesizing peptide or protein containing the mutation or the abnormal site, and extracting it from a tumor tissue.

In the present invention, the "X-peptide" refers to antigen peptide. Its examples are preferably, but are not limited to, tumor-related antigen such as GPC3, WT1, XAGE 1, LMP2, and neoantigen due to gene mutation and abnormality in splicing, more preferably GPC3.

The "T cells" are cells expressing antigen receptors called T cell receptors (TCRs) on the surface of cells. The T cells of the present invention are characterized by (i) having specificity to cancer vaccine antigen, (ii) being CD3 and CD45 positive, and (iii) producing IFN-$\gamma$. The term "having specificity to cancer vaccine antigen" means the binding/conjugation of T cells which are selective to major histocompatibility antigen (MHC) class I or class II to which cancer vaccine antigen or its decomposition products are bound, and reactions of T cells occurring due to the binding/conjugation, and refers to that binding/conjugation of T cells to major histocompatibility antigen class I or class II to which those other than target cancer vaccine antigen are bound is not allowed. The major histocompatibility antigen class I or class II to which the cancer vaccine antigen or its decomposition products are bound may be those expressed on cells, and may be a complex of the protein of the major histocompatibility antigen I or II to which cancer vaccine or its decomposition products are bound. Examples of the reactions of T cells occurring due to the binding/conjugation to major histocompatibility antigen (MHC) class I or class II to which cancer vaccine antigen or its decomposition products are bound include cytotoxicity, production of IFN-$\gamma$ and granzyme, and expression of T cell activation markers.

The term "T cells" used in the present invention includes T cell populations and regenerated T cell populations. A "T cell population" is a CD3 and CD45 positive lymphocyte, and a variety of cell populations wherein gene sequences of T cell receptors (TCRs) recognizing antigen present as a population. Therefore, T cells collected from a living body are T cell populations having specificity to various antigens. The T cells present in a living body have individually different TCR gene sequence due to random recombination of the TCR gene when T progenitor cells are generated and differentiated in thymus, and possibly cause immune response to any antigen. Although the TCR of each T cell is determined by antigen or peptide sequence that specifically recognizes each T cell, it can be understood that by considering T cells as a population, the T cell populations take various antigens as immune target. Therefore, T cells collected from a living body are those having genetic diversity

The term "regenerated T cell populations" refers to the T cell populations which are regenerated from a T cell population with genetic diversity collected from a living body via initialization to iPS cells, and the T cell populations which have directivity to an antigen while maintaining genetic diversity of the previous T cell populations before the initialization. The term "having directivity" refers to that individual cells indicate an immune response to a target tissue or antigen as a T cell population, while specifically recognizing different antigen epitopes or peptide sequences. Although iPS cells established from T cell populations having genetic diversity do not express TCR genes, they keep gene information of TCR produced by recombination in DNA, and maintain genetic diversity of T cell populations. Such iPS cells are cell populations wherein the gene sequences of TCR are different for individual cells.

In the present invention, T cells are preferably derived from mammals, and more preferably from human (patients with or without cancer). The patients with or without cancer are preferably those who had been administered, currently being administered, or scheduled to be administered a cancer vaccine. The cancer vaccine to be administered may be one or more types. Examples of T cells include $\alpha\beta$ T cells, $\gamma\delta$ T cells, helper T cells, cytotoxic T cells, and natural killer (NK) T cells, preferably cytotoxic T cells. In addition, as a sampling source of T cells, peripheral blood is preferred because of low invasiveness. However, it is not limited thereto. Other preferred sampling sources include all those from a living body such as cancer tissue, tumor tissue, or other tissue or organs, blood, umbilical cord blood, lymph, tissue liquids (inter-tissue liquid, inter-cell liquid, and interstitial fluid), body cavity liquid (abdominal liquid, pleural liquid, pericardial liquid, articular liquid, synovial fluid, and aqueous humor), nasal juice, urine, and the like. In an embodiment of the present invention, preferred T cells are those derived from tumor tissue, which are usually tumor infiltrated T cells.

Although there are various T cells in vivo so as to be able to response to any antigen, most of them are not related to the target antigen in T cell replenishment therapy. Therefore, it is effective in cancer treatment to concentrate the T cell populations reactive with a cancer vaccine antigen before use. On the other hand, when the concentrated T cell population is proliferated in order to obtain a large amount of T cells, the T cells are exhausted and immune response to the antigen is decreased. In order to solve this problem, it is preferrable to use regenerated T cell populations obtained by initializing the above concentrated T cell populations to iPS cells and inducing redifferentiation into T cells while maintaining genetic diversity of the previous T cell populations before the initialization.

The concentration of T cell populations is achieved by contacting with one or more types of cancer vaccine antigens to activate, separating and collecting the proliferated T cells. When T cells are brought into contact with a cancer vaccine antigen, T cells are activated by recognizing the cancer vaccine antigen by the T cell receptor and receiving the co-stimulation signal. As a method of contacting, for example, culture of mononuclear cells of peripheral blood containing antigen-presenting cells and T cells is performed using a culture medium added with a cancer vaccine antigen, a biological tissue containing a cancer vaccine antigen, a crushed product thereof, or a lysate thereof. It is possible to efficiently proliferate target T cells by adding cytokines such as IL-2, IL-7, IL-15, and IL-21 to a culture medium. The T cells which are cultured for proliferation express functional molecules such as CD137 molecules and IFN-$\gamma$ on the surface of cell membrane. T cells having directivity to the targeted cancer vaccine antigen can be concentrated by staining these functional molecules with a fluorescent protein or a specific antibody labeled with magnetic beads, and sorting using a flow cytometer or separating using a magnet. The concentration of T cells can also be performed using T cells collected from patients with cancer inoculated with cancer vaccines.

The concentration of T cells can also be performed as follows. In the process of culturing the mononuclear cells fraction containing antigen-presenting cells and T cells and adding the cancer vaccine antigen to the culture medium, the cancer vaccine antigen is repeatedly brought into contact with T cells so that the reactive T cells proliferate and the ratio to the whole T cell population increases. On the other hand, T cells that do not react with the added cancer vaccine antigen are not activated, their ratio in the T cell populations gradually decreases during *in vitro* culture, and they die away finally. Accordingly, after culturing for a certain period of time, a T cell population having diversity in the cancer vaccine antigen epitope or peptide sequence to be recognized while having a specific reactivity to the cancer vaccine antigen added into the culture medium can be concentrated and obtained.

As a method for concentrating T cell populations, a method of using T cells to which an oligomer of an antigen peptide-HLA (human leucocyte antigen) complex is bound can also be exemplified. In this method, an oligomer consisting of a complex of a specific HLA type and a target antigen peptide capable of presenting the HLA is labeled with a fluorescent dye, and cells to which the oligomer is bound are sorted using a flow cytometer, so that T cells having directivity to targeted tissue or antigen can be obtained. For concentrating T cell populations, these methods may be used alone or in combination.

The above concentrated T cell populations can be initialized to iPS cells and cultured while maintaining the genetic diversity of the concentrated T cell populations. Methods for producing iPS cells are well known in the art. The iPS cells are preferably produced by introducing an initialization factor into the concentrated T cell populations. Examples of the initialization factors include genes or gene products such as Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcll, deta-catenin, Lin28b, Sall1, Sal14, Esrrb, Nr5a2, Tbx3, or Glis1. These initialization factors can be used alone or in combination.

T cell populations present in a living body have a variety of TCR gene sequences as populations and have gene diversity in this sense. iPS cells established from the T cell populations are cultured while maintaining the genetic diversity. When the iPS cells are cultured, subculture is preferably carried out. However, in a general subculture method, since other cells except those to be used for subculture are discarded, the diversity of the iPS cells established from the T cell population having genetic diversity is gradually lost. In contrast, in the present invention, after establishing iPS cells, the culture vessel is preferably washed. Other cells except iPS cells are preferably removed. The iPS cells are recovered without cloning, and subcultured in another culture vessel. By repeating the subculture, iPS cells for redifferentiation into the regenerated T cell populations for use in the present invention can be obtained.

A culture medium used for culturing iPS cells is not particularly limited. It can be prepared by using a culture medium for culturing animal cells as a basic culture medium, and adding cytokines for maintaining capability of undifferentiation of iPS cells. Examples of a basic culture medium include culture media of Iscove's modified Dulbecco's medium (IMDM), Medium 199, Eagle's minimum essential medium (EMEM), $\alpha$MEM, Dulbecco's modified Eagle's medium (DMEM), Ham's F12, RPMI 1640, Fischer's, Neurobasal Medium (Life Technologies), StemFit® AK03N (Ajinomoto Healthy Supply), and a mixed culture medium thereof. Serum may be added to a culture medium, or a culture medium may be serum-free. As cytokines, bFGF can be preferably exemplified. The concentration thereof in a culture medium is, for example, 1 to 100 $\mu$g/mL (preferably 50 $\mu$g/mL).

The method for culturing iPS cells may be adhesion culture or suspension culture, preferably adhesion culture. As a method for separating iPS cells, for example, dynamical methods using a cell scraper or the like, and methods using a separation solution having protease activity, or collagenase activity, or both these activities (for example, Accutase® and Accumax®, etc.) can be exemplified.

IPS cells are preferably subcultured to another culture vessel when their cell density of $1 \times 10^3$ to $1 \times 10^4$ cells/cm², $1 \times 10^4$ to $1 \times 10^5$ cells/cm², or $1 \times 10^5$ to $1 \times 10^6$ cells/cm² is reached. The number of subcultures is not limited as long as the iPS cells are obtained at an amount required for T cell replenishment therapy, preferably 1 to 5 or 5 to 10.

The iPS cells having the genetic diversity obtained may be used as they are or cryopreserved until needed. Methods for cryopreservation of the cells are well known to those skilled in the art.

The regenerated T cell populations to be used in the present invention maintain the genetic diversity of the concentrated T cell populations described above and have directivity to cancer vaccine antigen used for activation of T cells.

The regenerated T cell populations to be used in the present invention are preferably obtained from the iPS cells cultured by differentiating into CD4/CD8 double-positive T cells via hematopoietic progenitor cells or from the iPS cells cultured by differentiating into CD8 single-positive T cells via hematopoietic progenitor cells and CD4/CD8 double-positive T cells.

Hematopoietic progenitor cells (HPCs) are cells capable of differentiating into blood cells such as lymphocytes, eosinophils, neutrophils, basophils, erythrocytes, or megakaryocytes. Hematopoietic progenitor cells and hematopoietic stem cells are not distinguished and refer to the same cells unless otherwise indicated. Hematopoietic stem cells/progenitor cells are recognized, for example, by that surface antigens CD34 and/or CD43 are positive.

Hematopoietic progenitor cells are preferably produced by culturing iPS cells in a culture medium added with vitamin C. The term "vitamin C" refers to L-ascorbic acid and its derivatives. An "L-ascorbic acid derivative" means a substance which converts into vitamin C by an enzymatic reaction *in vivo.* As examples of L-ascorbic acid derivatives, phosphate vitamin C, ascorbic acid glucoside, ascorbyl ethyl, vitamin C ester, tetrahexyldecanoic acid ascorbyl, ascorbyl stearate, and ascorbic acid-2 phosphoric acid-6 palmitic acid can be exemplified. The L-ascorbic acid derivative is preferably vitamin C. For example, a phosphoric acid-L ascorbate such as phosphoric acid-sodium L ascorbate or phosphoric acid-magnesium L ascorbate can be exemplified. A vitamin C is contained in a culture medium at a concentration of, for example, 5 to 500 μg/mL.

A culture medium used for producing hematopoietic progenitor cells is not particularly limited. It can be prepared by using a culture medium for culturing animal cells as a basic culture medium, and adding vitamin C or the like thereto. As a basic culture medium, culture media of Iscove's modified Dulbecco's medium (IMDM), Medium 199, Eagle's minimum essential medium (EMEM), αMEM, Dulbecco's modified Eagle's medium (DMEM), Ham's F12, RPMI 1640, Fischer's medium, Neurobasal Medium (Life Technologies), StemPro34 (Life Technologies), and a mixed culture medium thereof can be exemplified. A culture medium may contain serum or it may be serum-free. If necessary, a basic culture medium may contain one or more substances selected from albumin, insulin, transferrin, selenium, fatty acids, trace elements, 2-mercaptoethanol, thiol glycerol, lipids, amino acids, L-glutamine, non-essential amino acids, vitamins, growth factors, small molecular compounds, antibiotics, antioxidants, pyruvic acid, buffering agents, inorganic salts, cytokines, and the like.

A cytokine selected from the group consisting of BMP4 (bone morphogenetic protein 4), VEGF (vascular endothelial growth factor), bFGF (basic fibroblast growth factor), SCF (stem cell factor), TPO (thrombopoietin), and FLT-3L (Flt3 ligand) may be furtherly added to a culture medium used for producing hematopoietic progenitor cells. Their concentrations are, for example, 1 to 100 ng/mL for BMP4, 1 to 100 ng/mL for VEGF, 1 to 100 ng/mL for bFGF, 10 to 100 ng/mL for SCF, 1 to 100 ng/mL for TPO, and 1 to 100 ng/mL for FLT-3L.

A TGF β inhibitor may be added to the culture medium of hematopoietic progenitor cells. The term "TGF β inhibitor" refers to a small-molecular inhibitor interfering with the signaling of TGF β family, for example, SB431542 and SB202190 (R. K. Lindemann et al., Mol. Cancer 2: 20(2003), SB505124 (GlaxoSmithKline), NPC30345, SD093, SD908, and SD208 (Scios), and LY2109761, LY364947, and LY580276 (Lilly Research Laboratories). It can be added to a culture medium at a concentration of preferably 0.5 to 100 μM.

The iPS cells can be co-cultured with feeder cells such as C3H10T1/2 (Takayama N. et al. J Exp Med. 2817-2830, 2010), or stroma cells derived from different types (Niwa A. et al. J Cell Physiol. 2009 Nov; 221 (2): 367-77). However, preferably they may be cultured without using feeder cells.

Methods for culturing iPS cells in the production of hematopoietic progenitor cells can be adhesion culture or suspension culture, preferably suspension culture. For example, iPS cells can be subjected to suspension culture after separating the cultured colonies until 80% confluent to the used dish and dissociating the colonies into single cells. As a method for separating iPS cells, dynamical methods using a cell scraper or the like, and methods using a separation solution having protease activity and collagenase activity (for example, Accutase® and Accumax®, etc.) or a separation solution having collagenase activity can be exemplified.

The term "suspension culture" refers to a culture performed under a state where cells are not adhesive to culture vessel. Suspension culture can be performed, but is not particularly limited thereto, by using a culture vessel which is not artificially treated for improving adhesion to cells (for example, coating treatment by an extracellular matrix or the like), or a culture vessel which is artificially treated to suppress the adhesion (for example, coating treatment by polyhydroxyethyl methacrylate (poly-HEMA) or coated with nonionic surface-active polyol (such as Pluronic F-127). In case of suspension culture, it is preferable to form an embryoid body (EB) and perform the culture.

Hematopoietic progenitor cells can also be prepared from net-like structures (also referred to as iPS-sac) obtained by culturing iPS cells. The term "net-like structure" refers to a three-dimensional bursal structure (with internal space) derived from iPS cells, formed by endothelial cell populations or the like, and containing hematopoietic progenitor cells in the interior thereof.

The condition of temperature during the culture for producing hematopoietic progenitor cells from iPS cells is not particularly limited. However, it is, for example, about 37°C to about 42°C, preferably about 37°C to about 39°C. In addition, a person skilled in the art can appropriately determine the culturing period while monitoring the number of cells of hematopoietic progenitor cells or the like. The number of days for the culturing is not particularly limited as long as the hematopoietic progenitor cells are obtained, but is, for example, at least 6 days or more, 7 days or more, 8 days or more, 9 days or more, 10 days or more, 11 days or more, 12 days or more, 13 days or more, or 14 days or more, and preferably 14 days. A long culture period is not considered problematic in the production of hematopoietic progenitor cells. Besides, the culture may be performed under a condition of low oxygen, such as 15%, 10%, 9%, 8%, 7%, 6%, and 5% or less of oxygen concentration in an embodiment of the present invention.

The term "CD4/CD8 double-positive T cells" refers to cells ($CD8^+ CD4^+$) wherein both CD4 and CD8, the surface antigens are positive among T cells. Since T cells can be recognized by being positive of CD3 and CD45 which are surface antigens, the CD4/CD8 double-positive T cells can be identified as a cell being positive of CD4, CD8, CD3, and CD45. The CD4/CD8 double-positive T cells can be differentiated by induction into CD4 single-positive cells or CD8 single-positive cells.

The CD4/CD8 double-positive T cells can be produced by a method comprising a step of culturing hematopoietic progenitor cells in a culture medium added with p38 inhibitor and/or SDF-1.

The term "p38 inhibitor" is defined as a substance that inhibits the function of p38 protein (p38MAP kinase). As p38 inhibitors, chemical inhibitors of p38, dominant negative variants of p38, or nucleic acids encoding the same can be exemplified, however, they are not limited thereto.

Examples of chemical inhibitors of p38 include, but are not limited thereto, SB203580 (4-(4-fluorophenyl)-2-(4-methylsulfonylphenyl)-5-(4-pyridyl)-1H-imidazole) and its derivatives, SB202190 (4-(4-fluorophenyl)-2-(4-hydroxyphenyl)-5-(4-pyridyl)-1H-imidazole) and its derivatives, SB239063 (trans-4-[4-(4-fluorophenyl)-5-(2-methoxy-4-pyrimidinyl)-1H-imidazol-1-yl]cyclohexanol) and its derivatives, SB220025 and its derivatives, PD169316, RPR200765A, AMG-548, BIRB-796, SC10-469, SCIO-323, VX-702, or FR167653. These compounds are commercially available. For example, SB203580, SB202190, SC239063, SB220025 and PD169316 are available from Calbiochem, and SCI0-469 and SCIO-323 are available from Scios. A p38 inhibitor is added to the culture medium in a concentration, for example, about 1 μM to about 50 μM.

As dominant negative variants of p38, p38T180A in which the threonine at position 180 located in the DNA binding region of p38 is point-mutated to alanine, and p38Y182F in which the tyrosine at position 182 of p38 in human and mice is point-mutated to phenylalanine, and the like can be exemplified.

SDF-1 (stromal cell-derived factor 1) is not only SDF-1α or its mature form, but may also be an isoform such as SDF-1β, SDF-1γ, SDF-1δ, SDF-1ε, or SDF-1φ, or a mature form thereof, or a mixture of any ratio thereof. SDF-1α is preferably used. The SDF-1 may also be referred to as CXCL-12 or PBSF.

SDF-1 may be substituted, deleted and/or added with one or several amino acids in its amino acid sequence, as long as having the activity as chemokine. Similarly, its sugar chain may be substituted, deleted, and/or added. In SDF-1, if at least four cysteine residues (Cys30, Cys32, Cys55, and Cys71 in case of human SDF-1α) are maintained, and it has identity of 90% or more to the amino acid sequence of natural form, amino acid mutation is tolerated. SDF-1 may be derived from a mammal such as human, or non-human mammal like monkey, sheep, bovine, horse, swine, dog, cat, rabbit, rat, or mouse. For example, the protein registered with GenBank accession number of NP_954637 can be used as human SDF-1α, and the protein registered with GenBank accession number of NP_000600 can be used as SDF-1β.

As SDF-1, commercially available products, those purified from nature ones, or those produced by peptide synthesis or genetic engineering techniques may be used. SDF-1 is added to a culture medium in a concentration of, for example, about 10 ng/mL to about 100 ng/mL.

A culture medium used for the production of CD4/CD8 double-positive T cells is not particularly limited. It can be prepared by using a culture medium used for culturing animal cells as a basic culture medium, and adding a p38 inhibitor and/or SDF-1, and preferably further vitamin C thereto. The vitamin C used in the production process of the CD4/CD8 double-positive T cells is, for example, as described above. The concentration of vitamin C is, for example, 5 to 200 μg/mL. As a basic culture medium, culture media of Iscove's modified Dulbecco's medium (IMDM), Medium 199, Eagle's minimum essential medium (EMEM), αMEM, Dulbecco's modified Eagle's medium (DMEM), Ham's F12, RPMI 1640, Fischer's Neurobasal Medium (Life Technologies), and a mixed culture medium thereof can be exemplified. A culture medium may be added with serum, or may be serum-free. If necessary, a basic culture medium may contain one or more substances selected from albumin, insulin, transferrin, selenium, fatty acids, trace elements, 2-mercaptoethanol, thiol glycerol, lipids, amino acids, L-glutamine, non-essential amino acids, vitamins, growth factors, small molecular compounds, antibiotics, antioxidants, pyruvic acid, buffering agents, inorganic salts, and cytokines etc.

A cytokine selected from the group consisting of SCF, TPO (thrombopoietin), FLT-3L, and IL-7 can be further added to a culture medium used for producing CD4/CD8 double-positive T cells. Their concentrations are, for example, 10 to 100 ng/mL for SCF, 10 to 200 ng/mL for TPO, 1 to 100 ng/mL for FLT-3L, and 1 to 100 ng/mL for IL-7.

When producing the CD4/CD8 double-positive T cells, hematopoietic progenitor cells may be cultured using feeder cells, but preferably, the culture is performed without using feeder cells.

The hematopoietic progenitor cells may be subjected to adhesion culture or suspension culture, but it is preferable to perform adhesion culture. In case of adhesion culture, a culture vessel may be coated for use. As a coating agent, Matrigel (Niwa A. et al. PLoS One. 6(7): e22261, 2011), collagen, gelatin, laminin, heparan sulfate proteoglycan, Retronectin, Fc-DLL4, or entactin, and a combination thereof can be exemplified.

When hematopoietic progenitor cells are obtained by suspension culture of embryoid body, it is preferable to dissociate them into single cells and then perform adhesion culture.

The condition of temperature during the culture of hematopoietic progenitor cells for producing CD4/CD8 double-positive T cells is not particularly limited, but preferably for example, about 37°C to about 42°C, and more preferably about 37°C to about 39°C. In addition, a person skilled in the art can appropriately determine a culture period while monitoring the number of CD4/CD8 double-positive T cells or the like. The number of days for the culturing is not particularly limited as long as CD4/CD8 double-positive T cells are obtained, but is, for example, at least 10 days or more, 12 days or more, 14 days or more, 16 days or more, 18 days or more, 20 days or more, 22 days or more, or 23 days or more, and preferably 23 days.

The resulting CD4/CD8 double-positive T cells may be isolated for use, or may be used as a cell population containing other cell species. In case of isolation, an index selected from the group consisting of CD4, CD8, CD3, and CD45 can be used. Regarding the isolation methods, those well known to a person skilled in the art can be used. For example, an isolation method using a flow cytometer by labeling with an antibody against CD4, CD8, CD3, or CD45, or a purification method using an affinity column wherein a desired antigen is immobilized can be exemplified.

The term "CD8 single-positive T cells" refers to cells ($CD8^+$ $CD4^-$) among T cells wherein their surface antigen CD8 is positive, and is also referred to as cytotoxic T cells. Since T cells can be recognized by that surface antigens CD3 and CD45 are positive, the CD8 single-positive T cells can be identified as cells of CD8, CD3, and CD45 positive, and CD4 negative.

CD8 single-positive T cells can be produced by a method comprising a step of culturing CD4/CD8 double-positive T cells in a culture medium added with an adrenocortical hormone agent.

The adrenocortical hormone agent is preferably a glucocorticoid or a derivative thereof, such as cortisone acetate, hydrocortisone, fludrocortisone acetate, prednisolone, triamcinolone, methylprednisolone, dexamethasone, betamethasone, or beclomethasone propionate. Preferably, the adrenocortical hormone agent is dexamethasone. Its concentration in a culture medium is, for example, 1 to 100 nM.

A culture medium used for producing CD8 single-positive T cells is not particularly limited. However, it can be prepared by using a culture medium for culturing animal cells as a basic culture medium, and adding an adrenocortical hormone agent thereto. As a basic culture medium, culture media of Iscove's modified Dulbecco's medium (IMDM), Medium 199, Eagle's minimum essential medium (EMEM), αMEM, Dulbecco's modified Eagle's medium (DMEM), Ham's F12, RPMI 1640, Fischer's Neurobasal Medium (Life Technologies), and a mixed culture medium thereof can be exemplified. A culture medium may be added with serum, or may be serum-free. If necessary, a basic culture medium may contain one or more substances selected from albumin, insulin, transferrin, selenium, fatty acids, trace elements, 2-mercaptoethanol, thiol glycerol, lipids, amino acids, L-glutamine, non-essential amino acids, vitamins, growth factors, small molecular compounds, antibiotics, antioxidants, pyruvic acid, buffering agents, inorganic salts, and cytokines, etc.

A culture medium used in the production of CD8 single-positive T cells preferably further contains an anti-CD3 antibody, vitamin C, or cytokine. Examples of the cytokine include IL-2 and IL-7, etc.

The anti-CD3 antibody is not particularly limited as long as it is an antibody that specifically recognizes CD3, for example, an antibody produced from OKT3 clone. The concentration of the anti-CD3 antibody in a culture medium is, for example, 10 to 1000 ng/mL.

The vitamin C used in the production of CD8 single-positive T cells is, for example, as described above, and can be used under the same conditions as described above.

The concentration of a cytokine in a culture medium used in the production of CD8 single-positive T cells is, for example, 10 to 1000 U/mL for IL-2, and 1 to 100 ng/mL for IL-7.

The condition of temperature during the culture of CD4/CD8 double-positive T cells for the production of CD8 single-positive T cells is not particularly limited, but is, for example, preferably about 37°C to about 42°C, and more preferably about 37°C to about 39°C. In addition, a person skilled in the art can appropriately determine the culture period while monitoring the number of CD8 single-positive T cells or the like. The number of days for the culturing is not particularly limited as long as the CD8 single-positive T cells are obtained, but is, for example, at least 1 day or more, 2 days or more, 3 days or more, 4 days or more, or 5 days or more, and preferably 3 days.

In an embodiment of the present invention, the degree of genetic diversity to be maintained is preferably 30% or more, more preferably 40% or more, more preferably 50% or more of that of the T cell population having genetic diversity of the raw material. The degree of genetic diversity to be maintained can be confirmed, for example, based on repertoire analysis of TCR β gene using a TCRV β analysis kit (Beckman Coulter, catalog number:IM-3497). In an embodiment of the present invention, it was confirmed that the degree of genetic diversity was maintained at 40% or more of the T cell population having genetic diversity of the raw material, according to the repertoire analysis of the TCR β gene using the TCRV β analysis kit (Beckman Coulter, catalog number:IM-3497).

The survival rate of the regenerated T cells of the present invention is 60% or more, more preferably 70% or more, and even more preferably 80% or more. The survival rate is obtained by performing analysis with a cell counter, and calculating the ratio of the cells that do not incorporate a dye such as trypan blue that is specifically incorporated into dead cells.

The expression rate of CD3 of the regenerated T cells of the present invention is 70% or more, more preferably 80% or more, and even more preferably 90% or more. The expression rate of CD3 is calculated by using a flow cytometry.

In the regenerated T cells of the present invention, the expression ratio of exhaustion marker is 30% or less, more preferably 20% or less, and even more preferably 10% or less. The expression ratio of exhaustion marker of the regenerated T cells can be obtained by staining each T cell with antibodies (TIGIT-Pacific Blue, PD-1-APC/Cy7), and analyzing by a flow cytometer (BD FACSAria® II).

The regenerated T cells of the present invention have the capability to produce IFN-g/IL-2 (FIG. 15).

As a substance stimulating the regenerated T cells, a PMA/ION solution was prepared wherein the final concentration of PMA (Phorbol 12-Myristate 13 Acetate, WAKO) was 20 ng/mL and the final concentration of ION (Ionomycin, WAKO) was 1 μg/mL. The PMA/ION solution and Monensin (BioLegend) were added to the regenerated T cells and stimulated for 4 hours. After the stimulation, the surface antigen of the cells was stained with antibodies (CD45-BV510, CD8α-PerCP/Cy5.5, CD8β-APC, CD19-FITC), immobilized with Fixation Buffer (BioLegend), and permeated through a cell membrane. After the penetration, it was stained with cytokine antibodies (IFN-g-APC/Cy7, IL-2-PE), and the ratio of the regenerated T cells producing the cytokine was analyzed by using a flow cytometer (BD FACSAria® II). The ratios of the regenerated T cells (CD19-CD45+CD8a+CD8b+ cells) producing the cytokine in cases of with or without the stimulation of PMA/ION were compared, and the ratio of the cells producing both cytokines and each cytokine in the case of the stimulation was found to be significantly higher. These results suggest that the resulting regenerated T cells have the capability to produce IFN-g/IL-2.

The pharmaceutical composition of the present invention containing T cells as an active ingredient can be used for treating subjects with cancer. The pharmaceutical composition of the present invention can be produced by a conventional method in the technical field of formulation, for example, by a method described in the Pharmacopoeia of Japan. The pharmaceutical composition of the present invention may contain pharmaceutically acceptable additives. Examples of the additives include a cell culture medium, a physiological saline solution, and a suitable buffer solution (for example, phosphate buffer).

The pharmaceutical composition of the present invention can be produced by suspending T cells in a physiological saline solution or a suitable buffer solution (for example, phosphate buffer). In order to exhibit a desired therapeutic effect, it contains preferably for example, $1 \times 10^7$ or more, more preferably $1 \times 10^8$ or more, and even more preferably $1 \times 10^9$ or more cells as the amount of one dose. The content of the cells can be appropriately adjusted in consideration of the sex, age, body weight, state of the affected part, the state of the cells, etc. of the subject to whom the composition is applied. In addition to T cells, the pharmaceutical composition of the present invention may also contain dimethyl sulfoxide (DMSO) and serum albumin for the purpose of protecting cells. Besides, an antibiotic may also be contained for the purpose of preventing bacteria from being mixed, and vitamins and cytokines etc. may also be contained for the purpose of promoting activation and differentiation of cells. Furthermore, the pharmaceutical composition of the present invention may further contain other components acceptable for formulation (for example, a carrier, an excipient, a disintegrant, a buffer, an emulsifier, a suspension, an analgesic agent, a stabilizer, a preservative, an antiseptic agent, and physiological saline, etc.).

The pharmaceutical composition of the present invention containing T cells as an active ingredient can be cryopreserved at a low temperature. In the case of cryopreservation at a low temperature, the temperature is not particularly limited as long as it is suitable for cell preservation. For example, -20°C, -80°C, and -120 to -196°C can be mentioned. In the case of cryopreservation at a low temperature, the cells can be stored in an appropriate container such as a vial. Procedures for minimizing the risk of cell damage during freezing and thawing of T cells are well known to those skilled in the art.

In the cryopreservation of T cells, T cells are recovered from a culture medium, washed with a buffer solution or a culture medium, counted for the number of cells, concentrated with centrifugation or the like, suspended in a frozen medium (for example, a culture medium containing 10% DMSO), and then cryopreserved at a low temperature. Cells cultured in a plurality of culture vessels can be combined and used as a single lot. The pharmaceutical composition of the present invention contains for example, $5 \times 10^4$ to $9 \times 10^{10}$ T cells per container such as a vial. However, it can be changed according to the type of targeted cancer, the subject to be administered, and the administration route, etc.

The administration route of the pharmaceutical composition of the present invention containing T cells may be, for example, infusion, intratumor injection, arterial injection, portal vein injection, intraperitoneal administration, and the like. However, as long as T cells which are active ingredients in the pharmaceutical composition of the present invention are delivered to the affected part, the administration route is not limited thereto. As administration schedule, it is possible to administer a single time or a plurality of times. The period of a plurality of times of administration can be, for example, repeating the administration once for 2 to 4 weeks or once for half to one year. In the preparation of administration schedule can be prepared in consideration of sex, age, body weight, and pathological condition, etc. of the subject patient.

In the case where the pharmaceutical composition of the present invention containing T cells is used for the prevention or treatment of cancer, from the viewpoint of lower possibility of causing a rejection reaction in the case of other house transplantation, it is preferable that a patient with or without cancer from whom T cells are collected match a patient with cancer to whom the pharmaceutical composition of the present invention is administered for cancer treatment in their HLA type. In addition, it is more preferable that a patient with cancer to whom the pharmaceutical composition of the present invention is administered and a patient with cancer from whom the T cells are collected are the same person. That is, it is more preferable to treat cancer by autologous transplantation than by other-house transplantation.

The pharmaceutical composition of the present invention is used for the prevention or treatment of cancer. Examples of cancer include, but are not limited thereto, ovarian cancer, hepatoblastoma, hepatocellular cancer, gastric cancer, esophageal cancer, pancreatic cancer, renal cell cancer, breast cancer, malignant melanoma, non-small cell lung cancer, cervical cancer, glioblastoma, prostate cancer, neuroblastoma, chronic lymphocytic leukemia, colorectal cancer, colon cancer, and B-cell non-Hodgkin's lymphoma.

When the pharmaceutical composition of the present invention containing T cells and a cancer vaccine are used in combination for the prevention or treatment of cancer, their administration time is not limited, and both may be administered simultaneously or at a time difference. The pharmaceutical composition of the present invention and a cancer vaccine may be separately formulated or may be a mixture in which both are mixed. The dosage of the pharmaceutical composition of the present invention and a cancer vaccine may be a clinically used dose or may be appropriately selected according to a combination of disease, subject to be administered, administration route, and drugs.

The dosage form of the pharmaceutical composition of the present invention and a cancer vaccine is not particularly limited, as long as both are finally administered. Examples of such dosage forms include, but are not limited thereto, (1) administration of a single formulation obtained by formulating the both as one, (2) simultaneous administration of the both through the same route, (3) separate administration with a time difference through the same route, (4) simultaneous administration through different routes, (5) separate administration with a time difference through different routes (for example, administering a cancer vaccine, and then administering the pharmaceutical composition of the present invention, or administering in the reverse order).

By combining the pharmaceutical composition of the present invention and a cancer vaccine, the following excellent effects can be achieved.

(1) Compared with a case where the pharmaceutical composition of the present invention or a cancer vaccine is administered alone, the dosage can be reduced.
(2) Based on the administration of the pharmaceutical composition of the present invention and a cancer vaccine, a long treatment period can be scheduled.
(3) Based on the administration of the pharmaceutical composition of the present invention and a cancer vaccine, the therapeutic effect can be sustained.
(4) Based on combination of the pharmaceutical composition of the present invention and a cancer vaccine, synergistic anticancer or antitumor effects can be achieved.

In an embodiment of the present invention, T cells can be obtained by isolating and proliferating cytotoxic T cells against GPC3 from peripheral blood of a patient with hepatoblastoma administered with a cancer peptide vaccine (for example, cancer vaccine containing GPC3). Since the resulting T cells maintain antigen specificity to GPC3 and have antigen-specific cytotoxic activity to cancer cells (tumors) expressing GPC3, a pharmaceutical composition containing effective T cells can be obtained accordingly.

In an embodiment of the present invention, regenerated T cells can be obtained by isolating and proliferating cytotoxic T cells against GPC3 from peripheral blood of a patient with hepatoblastoma administered with a cancer peptide vaccine (for example, cancer vaccine containing GPC3), initializing the isolated and proliferated cytotoxic T cells into iPS cells, and then inducing redifferentiation from the iPS cells to cytotoxic T cells. Since the resulting regenerated T cells maintain antigen specificity to GPC3 and have antigen-specific cytotoxic activity to cancer cells expressing GPC3, a pharmaceutical composition containing effective regenerated T cells can be obtained accordingly.

The present invention will be described more specifically in the following examples. However, the scope of the present invention is not limited to these examples.

**EXAMPLES**

Method for producing cell products of the present invention

A method for producing cell products of the present invention is shown in FIG. 11.

The cell products of the present invention are produced through step (1) to step (15) shown in FIG. 11.

(1) Administer a cancer vaccine (X peptide + IFA) to a patient and induce an immune response to antigen X.

(2) Collect peripheral blood from the patient and separate mononuclear cells.

(3) Stimulate the resulting mononuclear cells with X-peptide in a test tube, proliferate and isolate the T cells specific to the antigen X.

(4) Introduce Yamanaka-four factors and SV40T antigen into the resulting T cells specific to the antigen X by using Sendai virus vector and perform initialization.

(5) Culture for proliferation of the established iPS cells while containing many clones, and produce a primary frozen stock.

(6) Produce a secondary frozen stock from the primary frozen stock.

(7) Thaw the secondary frozen stock, and culture for proliferation.

(8) After the culture for proliferation, induce the formation of endoderm containing blood cells by differentiation culture.

(9) Fractionate hematopoietic stem cells from the induced endoderm by using a flow cytometer.

(10) Culture the resulting hematopoietic stem cells together with T cell differentiation inducing factor to obtain immature T cells.

(11) Freeze and store the cell population at this point of time temporarily. Repeat the procedures so far to ensure sufficient amount of immature T cells.

(12) Freeze and thaw the frozen stock of the immature T cells, and start culture.

(13) Fractionate CD8$\alpha$/CD8$\beta$ double-positive T cells by a flow cytometer.

(14) Culture for maturation of the resulting cells with PHA and autologous feeder cells or feeder cells of other house.

(15) After the culture, wash the T cells, and then cryopreserve them for treatment.

Obtaining cells as raw material

Sensitization by administration of X peptide and IFA (FIG. 11(1)): A cancer vaccine (X peptide + IFA) was administered to a patient to promote cell proliferation of T cells that respond specifically to the X peptide *in vivo.* Peripheral blood was collected from the patient, and mononuclear cells were isolated (IFA: incomplete Freund's adjuvant).

(Step 1) Concentration of T cells

A cancer vaccine antigen (GPC3, WT1, XAGE 1, LMP2, or neoantigen) was added to a culture medium, brought into contact with a T cell population, and cultured for 12 days in a RPMI-1640 culture medium (including 10% human AB serum and 1% penicillin-streptomycin-glutamine) added with 200 U/mL IL-2, 10 ng/mL IL-15, and 10 ng/mL IL-21. In a case where the tumor-related antigen to be added the culture medium is a peptide, it is preferably added at a concentration of 1 to 100 mg/mL. In addition, in a case where a biological tissue or a crushed product thereof is used as a cancer vaccine antigen, tissue pieces shredded into 1 to 2 mm square are preferably added to a tube containing the culture medium at an amount of about 1 to 5 pieces per tube to bring it into contact with the T cell population. On day 12 of the culturing, the same cancer vaccine antigen was brought into contact again and cultured further for 24 hours to express the activation marker for T cells having directivity to the cancer vaccine antigen. Only CD8 single-positive T cells having directivity to the cancer vaccine antigens were concentrated by performing negative selection (CD8 + T Cell Isolation Kit, Miltenyi Biotech) with the cultured cell populations by a blood cell fraction marker except CD8 molecules and positive selection (CD137 MicroBead Kit or Cytokine capture system-IFN-y, Miltenyi Biotech) with an activation marker CD137 or IFN-$\gamma$ (FIG. 11(2)).

In a case where an antigen peptide-HLA complex is used in the above-described step, the T cell population to be used may be either a cell population that has been brought into contact with the cancer vaccine antigen and cultured for 12 days, or a cell population that is not cultured. A fluorescent labeled targeted antigen peptide-HLA complex was added to the resulting cell population, firstly stained for 30 minutes, then fluorescent labeled antibodies against other blood cell markers (CD8, CD4, and CD14) were added, and stained further for 30 minutes. After the staining, the collected cells were washed with a phosphate buffer solution, and then sorted only for the cells which express the antigen peptide-HLA complex and CD8 molecules with a flow cytometer to concentrate the T cells having directivity to the targeted cancer vaccine antigen (FIG. 11(3)).

(Step 2) Initialization to iPS cells (FIG. 11(4))

Subsequently, T cells were collected into a 15 mL tube, suspended in about 250 $\mu$L of RPMI-1640 culture medium. Then, Sendai virus containing four factors (Oct3/4, Sox2, Klf4, and c-Myc) necessary for initialization was added to the culture medium using CytoTune-iPS 2.0 kit (ID Pharma, catalog number: DV-0304) to achieve a titer of MOI=5, and incubated at 37°C for 2 hours. After the incubation, the T cells were washed using a RPMI-1640 culture medium to remove the virus from the culture medium.

Then, beads and the cells were separated by pipetting to remove the magnetic beads. Only the T cells suspended in the culture medium were collected by allowing the tube to stand on a stand with magnet.

The resulting T cells were seeded on a 6-cm dish coated with iMatrix-511 (Nippi, catalog number:892011) at 0.5 $\mu$g/cm$^2$, and started the culture in a RPMI-1640 culture medium using an incubator set at 37°C and 5% $CO_2$.

From the next day since the culture was started on the 6-cm dish, half of the culture supernatant containing no cells was removed. A culture medium for establishment added with 1 mM valproic acid was added to StemFit® AK03N (Ajinomoto Healthy Supply) at the same amount as that of the removed culture medium.

After that, the culture was continued for 20 to 40 days while exchanging half-amount of the culture medium every day in the same way, and a large number of iPS cell colonies were obtained in the 6-cm dish. When a certain amount of the colonies was obtained, the culture medium was replaced from that for establishment to that for iPS cells (only StemFit AK03N) and the culture was continued.

(Step 3) Culture while maintaining the genetic diversity of the established iPS cells (FIG. 11(5))

When the iPS cell colonies had grown big enough to be confirmed with naked eyes, the iPS cells were subcultured to a 6-cm dish newly coated with iMatrix-511.

In the subculture, the iPS cells adhered to the dish were washed with PBS (-), then added with TrypLeSelect (Life Technology, catalog number: A12859-01), and incubated for about 7 minutes to peel the iPS cells off and disperse into single cells. The cells were washed with the culture medium for iPS cells, and the number of the cells was counted. Then, the dispersed cells were seeded at a density of 1500 cells/cm$^2$ in a 6-cm dish newly coated with iMatrix-511. The collected iPS cells were all seeded into a new dish without being discarded.

By planting all collected cells, the culture scale was gradually expanded. When the subculture was performed 3 to 5 times after establishment, and enough amount of iPS cells were obtained for T cell replenishment therapy using the iPS cells, the cells were cryopreserved according to following procedures.

After the iPS cells were collected according to the same procedure as that for the subculture, the cells were suspended in a liquid for freezing cells such as TC Protector (DS Pharma, catalog number: KBTCP001), and the temperature was decreased at a speed of -1°C/minute to -80°C where the iPS cells were frozen. Furthermore, in order to keep the iPS cells stable for a long period of time, preservation in liquid nitrogen (-196°C) is preferable (FIG. 11(6)).

(Step 4) Production of regenerated T cell populations (FIG. 11(8))

The cryopreserved T-iPS cells were thawed, and cultured for proliferation (FIG. 11(7)).

The iPS cells obtained in the step 3 were seeded at $3\times10^5$ to $6\times10^5$ cells/well in a 6-well plate (Corning, catalog number:3471) treated for ultra-low adhesion (day 0). 10 ng/mL BMP4, 5 ng/mL bFGF, 15 ng/mL VEGF, and 2 $\mu$M SB431542 were added to an EB culture medium (StemPro34 containing 10 $\mu$g/mL human insulin, 5.5 $\mu$g/mL human transferrin, 5 ng/mL sodium selenate, 2 mM L-glutamine, 45 mM $\alpha$-monothioglycerol, and 50 $\mu$g/mL ascorbic acid), and cultured under low oxygen condition (5% $O_2$) for 5 days (day 5).

Subsequently, 50 ng/mL SCF, 30 ng/mL TPO, and 10 ng/mL Flt3L were added, and further cultured for 5 to 9 days (up to day 14) (FIG. 11(9)).

The resulting hematopoietic progenitor cells were cultured for 21 days (day 35) on a 4-well plate coated with Fc-DLL4 (5 $\mu$g/mL) (Sino Biological Inc.) and Retronectin (5 $\mu$g/mL) (Takara Bio) in an $\alpha$MEM culture medium (containing 15% FBS, 2 mM L-glutamine, 100 U/mL penicillin, 100 ng/mL streptomycin, 55 $\mu$M 2-mercaptoethanol, 50 $\mu$g/mL ascorbic acid, 10 $\mu$g/mL human insulin, 5.5 $\mu$g/mL human transferrin, and 5 ng/mL sodium selenite) added with 50 ng/mL SCF, 50 ng/mL IL-7, 50 ng/mL Flt3L, 100 ng/mL TPO, and 15 $\mu$M SB203580 (Tocris Bioscience) and 30 ng/mL SDF-1$\alpha$ (PeproTech) (FIG. 11(10)).

Cryopreservation of immature lymphocytes: The immature lymphocytic cells induced by the above-mentioned culture were collected, and cryopreserved using a liquid for freezing cells such as TC Protector (FIG. 11(11)).

The cryopreserved immature lymphocytic cells were thawed (FIG. 11(12)). On day 35, a fraction of CD4/CD8 double-positive cells (DP cells) being all of the CD45, CD3, CD4, and CD8 positive, or a fraction of CD8α/CD8β double-positive cells being all of the CD45, CD3, CD8α, and CD8β all positive was obtained using a flow cytometer (FIG. 11(13)).

The CD4/CD8 double-positive cells obtained from the immature lymphocytic cells induced by the above-mentioned culture were cultured on a 96-well plate for 2 days (day 37) in a RPMI 1640 culture medium added with 15% fetal bovine serum, 500 ng/mL anti-CD3 antibody (eBioscience), 200 U/mL Il-2, 10 ng/mL IL-7, and 10 nM dexamethasone. The cells were washed with a RPMI 1640 culture medium added with 15% fetal bovine serum to remove the antibody, and further cultured for 5 days in a RPMI 1640 culture medium added with 15% fetal bovine serum and 10 ng/mL IL-7. CD8 single positive T cells were obtained (day 42). The CD8α/CD8β double-positive cells from the immature lymphocytic cells induced by the above-mentioned culture were cultured on a 96-well plate for 21 days in an αMEM culture medium added with 15% fetal bovine serum, 2 μg/mL phytohemagglutinin (PHA), 40Gy radiation-irradiated mononuclear cells of peripheral blood (PBMC), 200 U/mL IL-2, 10 ng/mL IL-7, 5 ng/mL IL-15, 5 ng/mL IL-21,50 μg/mL ascorbic acid, 2 mM L-glutamine, 100 U/mL penicillin, 100 ng/mL streptomycin, a mixed solution of insulin, transferrin, and selenium (Thermo Fisher Scientific: ITS-G solution, catalog number:41400045). The mature CD8α/CD8β double-positive T cells were obtained (FIG. 11(14)).

Washing and freezing the mature T cells

The mature T cells obtained in the previous step were proliferated, washed with PBS, and then cryopreserved using a liquid for freezing cells such as TC Protector (FIG. 11(15)).

Example 1

According to the above method, iPS cells were established from a T cell population having genetic diversity separated from extirpated tumor of a patient with lung cancer, and induced to re-differentiate to T cells having the genetic diversity.

Although the genetic diversity of a T cell receptor (TCR) expressed by the T cells is generally determined by sequence analysis of DNA or RNA, it can also be easily detected by using an antibody panel specifically binding to each segment of Vβ chain of the TCR.

The TCR repertoire of a T cell population separated from extirpated tumor of a patient with lung cancer was analyzed by using a TCR Vβ analysis kit (Beckman Coulter, catalog number:IM-3497) (FIG. 1). It was confirmed that cells expressing the respective Vβ chains were contained in the specimen of the patient at a ratio of about 1 to 13% and they were T cell populations having genetic diversity. These T cell populations were initialized to iPS cells according to the above-mentioned method.

iPS cells are pluripotent stem cells, and do not express the T cell-related gene including TCR gene. That is, it is difficult to analyze the diversity of TCR gene at the stage of undifferentiated iPS cells. The established iPS cells were subcultured so as not to lose genetic diversity, and then subjected to differentiation induction to T cells via a stage of hematopoietic progenitor cells.

The repertoire of TCR expressing the regenerated T cells was analyzed by using the kit described above (FIG. 2). It was confirmed that although the diversity of T cells expressing the segments of 2, 3, 4, 7.1, 7.2, 8, 12, 14, 16, 20, and 21.3 of Vβ was lost due to the establishment of iPS cells and the differentiation induction of T cells, about half of the Vβ repertoire was remained, and the regenerated T cells were the T cell populations having genetic diversity.

Example 2

Peripheral blood was collected from a patient into whom a peptide vaccine was administered against GPC3, a cancer antigen, and the fraction of mononuclear cells was separated by using density gradient centrifugation method. $2\times10^6$ cells of the cell population were added to a 24-well plate. T cells having directivity to GPC3 were cultured for proliferation according to step 1 described above using 10 mg/mL GPC3 peptide. The cultured cells were again brought into contact with the GPC3 peptide and cultured for 24 hours to express activation marker. The cell population was stained with antibodies (CD4-BV421, CD8-PerCP/Cy5.5, CD14-FITC, and CD137-PE). The ratio of the T cell population having GPC3 directivity was analyzed by using a flow cytometer (BD FACSAria® II) (FIG. 3). As a result, it was shown that the CD8/CD137 double-positive cells having directivity to GPC3 were proliferated at a high ratio. For this cell population, the T cell population which expresses both CD8 and CD137 and has directivity to GPC3 was concentrated by using magnetic beads (FIG. 4). The cell population was initialized to iPS cells according to steps 2 and 3 described above, and then induced to redifferentiation to CD8 single-positive cells according to step 4 described above. The re-differentiated CD8 positive cells were stained with antibodies (CD3-APC/Cy7, CD4-BV421, CD8a-PerCP/Cy5.5, CD8b-PE, TCRab-FITC, CD45-BV510, and GPC3-HLA complex (Dextramer)-APC) and analyzed using a flow cytometer (BD FACSAria® II) (FIG. 5). As shown by the results, the re-differentiated CD8-positive cells were not expressing CD4. They were CD8a/CD8b double-positive, and bound to GPC3 dextramer at a high ratio (FIG. 5).

**Example 3**

A T cell population having directivity to WT1 was cultured for proliferation in the same manner as that in Example 2 by separating mononuclear cells from peripheral blood of a patient with lung cancer and bringing into contact with an overwrap peptide of WT1 (PepTivator WT1, Miltenyi Biotech), a tumor antigen. The overwrap peptide of WT1 was added to the culture medium at a concentration of 1 $\mu$g/mL. The activation marker was expressed by bringing the cultured cells again into contact with the overwrap peptide of WT1. As shown by the results of the analysis, the CD8/CD137 double-positive cells having directivity to WT1 were proliferated at a high ratio (FIG. 6). For this cell population, the T cell population expressing both CD8 and CD137 and having directivity to WT1 was concentrated by using magnetic beads (FIG. 7).

**Example 4**

A T cell population having directivity to EBV LMP2A was cultured for proliferation in the same manner as that in Example 2 by separating mononuclear cells from peripheral blood of a patient without cancer (healthy subject) and bringing into contact with an overwrap peptide of EBV LMP2A (PepTivator EBV LMP2A, Miltenyi Biotech), a virus antigen. The overwrap peptide of EBV LMP2A was added to the culture medium at a concentration of 1 $\mu$g/mL. The activation marker was expressed by bringing the cultured cells again into contact with the overwrap peptide of EBV LMP2A. As shown by the results of the analysis, the CD8/CD137 double-positive cells having directivity to EBV LMP2A were proliferated at a high ratio (FIG. 8). For this cell population, the T cell population expressing both CD8 and CD137 and having directivity to EBV LMP2A was concentrated by using magnetic beads (FIG. 9).

**Example 5**

A regenerated T cell population produced via iPS cells from a T cell population having directivity to GPC3 was confirmed to have specific cytotoxic activity to target cells expressing the GPC3 peptide.

A T cell population having directivity to GPC3 was cultured for proliferation by separating mononuclear cells from peripheral blood of a patient with liver cancer, and bringing into contact with GPC3 peptide in the same manner as that in Example 2, and further concentrated. The concentrated cell population was initialized to iPS cells according to the above steps 2 and 3, and then introduced to redifferentiation to CD8 single-positive cells according to the above step 4. From the analysis with a flow cytometry of the resulting regenerated T cell population, it was confirmed that CD8 was present as CD8$\alpha\beta$ heterodimer (FIG. 10A).

B cells infected and immortalized with EB virus were used as target cells. The target cells were cultured in the presence (1 $\mu$M) or absence of GPC3 peptide. Cells which express GPC3 peptide and cells which do not express GPC3 peptide were produced. Cytotoxic activity against the target cells of the regenerated T cell population was determined by using an N-SPC non-RI cytotoxic assay kit (Techno Suzuta). The cells which express GPC3 peptide and cells which do not express GPC3 peptide were treated with a BM-HT reagent and incorporated with the BM-HT reagent. Then, the target cells were washed to remove the excess BM-HT reagent. The target cells and the regenerated T cell population were co-cultured for 2 hours. Its culture supernatant was added to an Eu (europium) solution. The HT chelate leaking into the culture medium from the damaged target cells turned into HT/EU complex, which was excited by a laser beam, and assessed for cytotoxic activity by measuring time-resolved fluorescence. The cytotoxic activity was calculated by the following equation:

$$\text{Cytotoxic activity (\%)} = [\text{fluorescence amount in the culture supernatant in the presence of regenerated T cells - fluorescence amount in the culture supernatant in the absence of the regenerated T cells}] / [\text{fluorescence amount in the solution where all target cells are dissolved - fluorescence amount in the culture supernatant in the absence of the regenerated T cells}] \times 100$$

The ratio of the cell numbers of the regenerated T cells to the target cells in the co-culture was taken as 20:1, 10:1, and 5:1. As a result, the cytotoxic activity of the regenerated T cell population to target cells expressing the GPC3 peptide was indicated, and the activity enhanced in response to an increase in the cell number of the regenerated T cells with respect to the target cells (FIG. 10B). On the other hand, the regenerated T cell population showed no cytotoxic activity to the target cells which did not express the GPC3 peptide (FIG. 10B). As shown by these results, the resulting regenerated T cell population maintains antigen specificity to GPC3 and has antigen-specific cytotoxic activity to target cells expressing GPC3.

**Example 6**

An example of the method for producing the cell products of the present invention

An example of the method for producing the cell products of the present invention is shown in FIG. 12.

A process for producing CD8α/CD8β double-positive regenerated T cells from iPS cells derived from tumor antigen X-reactive T cells (tumor-specific T cells) is shown. The differentiation induction of hematopoietic stem cells is performed from the iPS cells derived from the tumor antigen X-reactive T cells using an embryoid body formation method, and CD34/CD43 double-positive hematopoietic stem cells are isolated with a flow cytometer. The resulting CD34/CD43 double-positive hematopoietic stem cells are cultured together with T cell differentiation inducer (T cell induction) to induce differentiation of immature T cells. The CD8α/CD8β double positive immature T cells induced are isolated by using a flow cytometer, and cultured together with PHA and feeder cells to give mature T cells. The CD3-positive mature T cells are tumor antigen X-reactive. (The data of a typical example are shown. The numerals in the figures indicate appearance frequency (%) thereof.)

**Example 7**

Specification of the cell products of the present invention

Targeted values of the specification of the cell products of the present invention are shown in FIG. 13.

The targeted values of the specification of the products are set as follows. The outlines of various tests are as follows. (1) Sterility test: a test for denying the presence of bacteria in product packages, carried out by using the direct method or the membrane filter method of the 17th revision of Pharmacopoeia of Japan, and microorganism rapid assay (BACT/ALERT), etc. The results should be confirmed as being negative. (2) Virus test: a test for denying the presence of human virus exhibiting pathogenicity in humans, such as HIV-1, HIV-2, HBV, HCV, HTLV, ParvoB19, EBV, CMV, and WNV, in the cell products. The results should be confirmed as being negative. (3) Mycoplasma test: a test for denying the absence of mycoplasma infection in the cell products, carried out by using the culturing method, DNA staining method, and NAT method according to the reference information of the 17th revision of Pharmacopoeia of Japan. The results should be confirmed as being negative. (4) Endotoxin test: a test for denying the Gram-negative bacterial components in cell wall, carried out by using kinetics nephelometry of the 17th revision of Pharmacopoeia of Japan, or the like. The results should be confirmed as being not higher than the detection limit. (5) STR (short tandem repeat) test: a test for confirming that a cell product is derived from a patient, for confirming identity, and for detecting cross contamination in a product, where the short tandem repeat in the gene is detected as uPCR to confirm genotype. It should be confirmed that the STR genotype of a product produced matches that of the patient's cells at the time of acceptance. (6) Cell count: analyzed by using a cell counter. A total number of cell count in a product should be confirmed to be $1.5 \times 10^9$ or more. (7) Survival rate: analyzed by using a cell counter. The ratio of the cells not incorporating the dye such as trypan blue specifically incorporated into dead cells is detected. A survival rate should be confirmed as being 80% or more. (8) Survival rate after freezing and thawing: Although the products are shipped as frozen cell preparations, their survival rates after thawing should be confirmed in advance as being 80% or more. (9) Cell morphology: observed by using a microscope, and should be confirmed to be lymphocyte-like. (10) Karyotype detection: carried out by simple karyotype analysis (Q-band analysis), G-band analysis, and FISH analysis, etc. for conforming that there is no abnormality in the chromosome of a cell product. (11) Foreign substance detection: carried out with naked eyes for confirming that there is no foreign substance in the package of a product. (12) Test of residual Sendai virus: carried out by qPCR for validating that the Sendai virus used when introducing initialization factors does not remain in the cell products. The results should be confirmed as being not higher than the detection limit. (13) Analysis of T cell markers: for confirming that the cell products are T cells, carried out by analyzing expression of T cell markers, such as CD3 molecules, by using a flow cytometry. A ratio of the cells having high expression to the negative control should be confirmed to be 90% or more.

**Example 8**

Downregulation of exhaustion marker of the regenerated T cells of the present invention

The downregulation of exhaustion marker of the regenerated T cells of the present invention is shown in FIG. 14. The expression of the T cell populations having directivity to GPC3 cultured for proliferation according to step 1 (left) and cell exhaustion marker in CD8-positive cells induced for redifferentiation according to step 4 (right) were analyzed. Each cell was stained with antibodies (TIGIT-Pacific Blue, PD-1-APC/Cy7), and analyzed using a flow cytometer (BD FACSAria® II). While 70% of the proliferated T cell populations having directivity to GPC3 expressed either of the cell exhaustion markers, the expression ratio of the cell exhaustion marker in CD8-positive cells induced for redifferentiation was decreased to about 10%.

**Example 9**

The regenerated T cells of the present invention produce IFN-γ/IL-2 upon PMA/ION stimulation

The results that the regenerated T cells of the present invention have the capability to produce IFN-γ/IL-2 are shown in FIG. 15.

APMA/ION solution was prepared as a substance stimulating the regenerated T cells, wherein the final concentration of PMA (Phorbol 12-Myristate 13 Acetate, WAKO) was 20 ng/mL and the final concentration of ION (Ionomycin, WAKO) was 1 μg/mL. The PMA/ION solution and Monensin (BioLegend) were added to the regenerated T cells and stimulated for 4 hours. After the stimulation, the surface antigen of the cells was stained with antibodies (CD45-BV510, CD8α-PerCP/Cy5.5, CD8β-APC, and CD19-FITC), and immobilized with a Fixation Buffer (BioLegend), and permeated through a cell membrane. After the penetration, it was stained with cytokine antibodies (IFN-γ-APC/Cy7, IL-2-PE), and the ratio of the regenerated T cells producing the cytokine was analyzed using a flow cytometer (BD FACSAria® II). The ratios of the regenerated T cells producing the cytokine (CD19- CD45+ CD8a+ CD8b+ cells) were compared in cases of with and without the PMA/ION stimulation, and the ratio of the cells producing both cytokines and either cytokine in the case of with the stimulation was significantly higher. As suggested by the results, the resulting regenerated T cells have the capability to produce IFN-γ/IL-2.

**Claims**

1. A pharmaceutical composition containing T cells as active ingredient, wherein the T cells are **characterized by**

    (i) having specificity to cancer vaccine antigen
    (ii) being CD3 and CD45 positive, and
    (iii) producing IFN-γ.

2. The pharmaceutical composition according to claim 1, wherein the cancer vaccine antigen is one or more selected from the group consisting of GPC3, WT1, XAGE 1, LMP2, and neoantigens.

3. The pharmaceutical composition according to claim 1, wherein the cancer vaccine antigen is GPC3.

4. The pharmaceutical composition according to claim 1, wherein the T cells are αβ T cells, γδ T cells, helper T cells, cytotoxic T cells, natural killer (NK) T cells, or a mixture thereof.

5. The pharmaceutical composition according to claim 1, wherein the T cells are cytotoxic T cells.

6. The pharmaceutical composition according to claim 5, wherein the cytotoxic T cells are the T cells obtained by collecting T cells from a patient with cancer inoculated with a cancer vaccine, fractionating cytotoxic T cells for the cancer vaccine antigen contained in the cancer vaccine, and proliferating the fractionated cytotoxic T cells.

7. The pharmaceutical composition according to claim 6, **characterized in that** the cytotoxic T cells are fractionated from the T cells by using one or more steps selected from the group consisting of a step of selecting T cells which express CD137, a step of selecting T cells which produce IFN-γ, and a step of selecting T cells to which antigen peptide-HLA complex binds.

8. The pharmaceutical composition according to claim 6, **characterized in that** the cytotoxic T cells are proliferated in the presence of one or more cytokines selected from the group consisting of IL-2, IL-7, IL-15, and IL-21.

9. The pharmaceutical composition according to claim 5, wherein the cytotoxic T cells are regenerated T cell populations obtained by inducing redifferentiation from iPS cells to the cytotoxic T cells.

10. The pharmaceutical composition according to claim 9, wherein the iPS cells are produced from the T cells having specificity to the cancer vaccine antigen by bringing one or more cancer vaccine antigens selected from the group consisting of GPC3, WT1, XAGE 1, LMP2, and neoantigens into contact with the T cells.

11. The pharmaceutical composition according to claim 9, wherein the iPS cells are produced from the T cells having specificity to GPC3 by bringing the cancer vaccine antigen GPC3 into contact with the T cells.

12. The pharmaceutical composition according to claim 10 or 11, wherein the T cells are collected from the peripheral blood of a patient with cancer or a healthy subject.

13. The pharmaceutical composition according to claim 10 or 11, wherein the T cells are collected from the peripheral blood of a patient with cancer.

14. The pharmaceutical composition according to claim 9, wherein the redifferentiation comprises fractionating CD8$\alpha$/CD8$\beta$ double-positive T cells by using a flow cytometer.

15. The pharmaceutical composition according to claim 9, further comprising culturing for maturation of the re-differentiated T cells with phytohemagglutinin (PHA) and feeder cells of autologous or other house.

16. The pharmaceutical composition according to any one of claims 1 to 15, wherein the T cells are mature CD8$\alpha$/CD8$\beta$ double-positive T cells.

17. The pharmaceutical composition according to any one of claims 1 to 16, wherein the expression ratio of CD3 of the T cell is 70% or more.

18. The pharmaceutical composition according to any one of claims 1 to 17, wherein the T cells have the capability to produce IFN-$\gamma$/IL-2.

19. The pharmaceutical composition according to any one of claims 1 to 18, wherein the survival rate of the T cells is 60% or more.

20. The pharmaceutical composition according to any one of claims 1 to 19, further comprising a pharmaceutically acceptable additive.

21. The pharmaceutical composition according to claim 20, wherein the additive is one or more selected from the group consisting of a cell culture medium, a physiological saline solution, and a buffer solution.

22. The pharmaceutical composition according to any one of claims 1 to 21, **characterized by** being used in combination with a cancer vaccine.

23. The pharmaceutical composition according to any one of claims 1 to 21, **characterized by** being administered simultaneously with a cancer vaccine.

24. The pharmaceutical composition according to any one of claims 1 to 21, **characterized by** being administered before or after the administration of a cancer vaccine.

25. The pharmaceutical composition according to any one of claims 22 to 24, wherein the cancer vaccine antigen contained in the cancer vaccine is one or more selected from the group consisting of GPC3, WT1, XAGE 1, LMP2, and neoantigens.

26. The pharmaceutical composition according to any one of claims 22 to 24, wherein the cancer vaccine antigen contained in the cancer vaccine is GPC3.

27. The pharmaceutical composition according to any one of claims 1 to 26, containing $1 \times 10^6$ or more T cells as the amount of one dose.

28. An anticancer agent comprising the pharmaceutical composition according to any one of claims 1 to 27.

**29.** The pharmaceutical composition according to any one of claims 1 to 27 for prevention or treatment of cancer.

**30.** The pharmaceutical composition according to claim 29, wherein the cancer is selected from the group consisting of ovarian cancer, hepatoblastoma, hepatocellular cancer, gastric cancer, esophageal cancer, pancreatic cancer, renal cell cancer, breast cancer, malignant melanoma, non-small cell lung cancer, cervical cancer, glioblastoma, prostate cancer, neuroblastoma, chronic lymphocytic leukemia, colorectal cancer, colon cancer, and B-cell non-Hodgkin's lymphoma.

**31.** A method for preventing or treating cancer in a patient with cancer, **characterized by** administering an effective amount of the pharmaceutical composition according to claim 30 to the patient with cancer.

**32.** The method for preventing or treating cancer according to claim 31, wherein the administration is a single administration or multiple administrations.

Figure 1

Figure 2

Figure 3

Figure 4

Figure 5

Figure 6

Figure 7

Figure 8

Figure 9

Figure 10

(A)
Regenerated CD8 α β positive T cells

(B)

Each point indicates "average value ± standard deviation".

Figure 11A

Figure 11B

Figure 12

iPS cells made from tumor-specific T cells

Hematopoietic stem cells from iPS cells

Regenerated CD8αβ positive T cells (population) produced

The regenerated CD8 positive T cells (population) produced maintain specificity to tumor antigens.

Binding to tumor antigens

Figure 13

| Specification items | Product performance |
|---|---|
| Sterility test | Negative |
| Virus test | Negative |
| Mycoplasma test | Negative |
| Endotoxin test | Not higher than the detection limit |
| STR (short tandem repeat) test | match |
| Cell count | $1.5 \times 10^9$ or more |
| Survival rate | 80% or more |
| Survival rate after freezing and thawing | 80% or more |
| Cell morphology | Lymphocyte-like |
| Karyotype | No abnormality |
| Foreign substances | Not visible to naked eyes |
| Residual Sendai virus | Not higher than the detection limit |
| T cell markers (CD3) | 90% or more |

Figure 14

T cells before iPS reprogramming → T-IPSC-derived T cells

Figure 15

**INTERNATIONAL SEARCH REPORT**

| International application No. |
| --- |
| PCT/JP2020/023308 |

### A. CLASSIFICATION OF SUBJECT MATTER

A61K 39/00(2006.01)i; A61P 35/00(2006.01)i; A61K 35/17(2015.01)i; A61K 35/545(2015.01)i
FI: A61K35/17 Z; A61K39/00 H; A61K35/545; A61P35/00

According to International Patent Classification (IPC) or to both national classification and IPC

### B. FIELDS SEARCHED

Minimum documentation searched (classification system followed by classification symbols)
A61K39/00; A61P35/00; A61K35/17; A61K35/545

Documentation searched other than minimum documentation to the extent that such documents are included in the fields searched
Published examined utility model applications of Japan 1922–1996
Published unexamined utility model applications of Japan 1971–2020
Registered utility model specifications of Japan 1996–2020
Published registered utility model applications of Japan 1994–2020

Electronic data base consulted during the international search (name of data base and, where practicable, search terms used)
JSTPlus/JMEDPus/JST7580 (JDreamIII); CAplus/MEDLINE/EMBASE/BIOSIS/WPIDS (STN)

### C. DOCUMENTS CONSIDERED TO BE RELEVANT

| Category* | Citation of document, with indication, where appropriate, of the relevant passages | Relevant to claim No. |
| --- | --- | --- |
| X<br>Y | VIZCARDO, R. et al., "Regeneration of human tumor antigen-specific T cells from iPSCs derived from mature CD8(+) T cells", Cell Stem Cell, 2013, vol. 12, no. 1, pp. 31-6, summary, fig. 1, page 31, right column, paragraph [0001] | 1,4,5,9,14,<br>16-24,27-32<br>6-8,12,13,<br>15,18 |
| A | 塚本 博丈ほか，T細胞を介した抗腫瘍免疫応答、実験医学，2013, vol. 31, no. 12 (extra edition), pp. 32-39, section "3) Effector T cells and memory T cells", non-official translation (TSUKAMOTO, Hirotake et al., "Anti-tumor immune response via T cells", Experimental medicine) | 1-32 |
| Y | WO 2019/070021 A1 (THYAS CO., LTD.) 11.04.2019 (2019-04-11) paragraphs [0014]-[0076], examples | 6-8,12,13 |

☒ Further documents are listed in the continuation of Box C.    ☒ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 03 August 2020 (03.08.2020) | 18 August 2020 (18.08.2020) |

| Name and mailing address of the ISA/<br>Japan Patent Office<br>3-4-3, Kasumigaseki, Chiyoda-ku,<br>Tokyo 100-8915, Japan | Authorized officer |
| --- | --- |
| | Telephone No. |

Form PCT/ISA/210 (second sheet) (January 2015)

**INTERNATIONAL SEARCH REPORT**

| International application No. |
| --- |
| PCT/JP2020/023308 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | WO 2011/096482 A1 (THE UNIVERSITY OF TOKYO) 11.08.2011 (2011-08-11) claims 1-7, paragraph [0042], examples 1-6 | 15,18 |
| X | WO 2016/010155 A1 (KAWAMOTO, Hiroshi) 21.01.2016 (2016-01-21) paragraph [0010], examples 1, 3 | 1-5,9-12,14, 16-32 |
| Y | | 6-8,12,13,15,18 |
| X | WO 2017/221975 A1 (KYOTO UNIVERSITY) 28.12.2017 (2017-12-28) example 3 | 1-5,9-11,14, 16-32 |
| Y | | 6-8,12,13,15,18 |
| A | SAITO, Hidehito et al., "Reprogramming of Melanoma Tumor-Infiltrating Lymphocytes to Induced Pluripotent Stem Cells", Stem Cells International, 2016, vol. 2016, Article ID 8394960, abstract, supplementary material | 1-32 |
| P,X | WO 2020/027094 A1 (THYAS CO., LTD.) 06.02.2020 (2020-02-06) claims 1-22 | 1-32 |

Form PCT/ISA/210 (continuation of second sheet) (January 2015)

**INTERNATIONAL SEARCH REPORT**
Information on patent family members

International application No.

PCT/JP2020/023308

| Patent Documents referred in the Report | Publication Date | Patent Family | Publication Date |
|---|---|---|---|
| WO 2019/070021 A1 | 11 Apr. 2019 | (Family: none) | |
| WO 2011/096482 A1 | 11 Aug. 2011 | US 2013/0078226 A1 claims 1-7, paragraph [0160], examples A1-A6 US 2017/0009206 A1 | |
| WO 2016/010155 A1 | 21 Jan. 2016 | US 2017/0267972 A1 paragraph [0020], examples 1, 3 EP 3170895 A1 AU 2015290561 A | |
| WO 2017/221975 A1 | 28 Dec. 2017 | US 2019/0330596 A1 example 3 EP 3476934 A1 CN 109415699 A | |
| WO 2020/027094 A1 | 06 Feb. 2020 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017534261 A
- JP 2017530694 A
- JP 2018514204 A
- JP 2017158559 A

**Non-patent literature cited in the description**

- **NISHIMURA T et al.** Generation of rejuvenated antigen-specific T cells by reprogramming to pluripotency and redifferentiation. *Cell Stem Cell,* 2013, vol. 12, 114-126
- **R. K. LINDEMANN et al.** *Mol. Cancer,* 2003, vol. 2, 20
- **TAKAYAMA N. et al.** *J Exp Med,* 2010, 2817-2830
- **NIWA A. et al.** *J Cell Physiol,* November 2009, vol. 221 (2), 367-77
- **NIWA A. et al.** *PLoS One,* 2011, vol. 6 (7), e22261
- *Pharmacopoeia of Japan*